# EUROPEAN PATENT SPECIFICATION

(11) **EP 4 608 349 B1**
(45) Date of publication and mention of the grant of the patent: **31.12.2025**
(21) Application number: 24714778.8
(22) Date of filing: 07.02.2024
(51) Int. Cl.: A61G 3/06, G16H 40/00

(54) **ASSEMBLY AND METHOD FOR TRANSPORTING A WHEELCHAIR FOR TRANSPORTING PERSONS WITH DISABILITY**
ANORDNUNG UND VERFAHREN ZUM TRANSPORT EINES ROLLSTUHLS ZUM TRANSPORT VON BEHINDERTEN PERSONEN
ENSEMBLE ET PROCÉDÉ DE TRANSPORT D'UN FAUTEUIL ROULANT POUR LE TRANSPORT DE PERSONNES HANDICAPÉES

(30) Priority: 16.02.2023 CZ 20230058
(43) Date of publication of application: 03.09.2025
(73) Proprietor: CV Machining s.r.o., 76001 Zlin, Kudlov (CZ)
(72) Inventor: VANCURA, Cestmir, 76001 Zlin (CZ); VYSTRCILOVA, Vera, 25168 Kamenice (CZ)
(74) Representative: Tomickova, Dana
(86) International application number: PCT/CZ2024/050009
(87) International publication number: WO 2024/170016

(56) References cited:
- US-A- 4 664 584

## Description

### Technical Field

The present invention relates to adapting automobiles for transporting wheelchairs, and in particular relates to transferring a wheelchair to and from an automobile, where the user can be seated in the wheelchair during the transfer as well as during the travel in the automobile, particularly while driving.

### Background of the Invention

Persons dependent on wheelchairs for their transport are limited when using passenger automobiles using the usual method of transferring from the wheelchair to the automobile and from the automobile back to the wheelchair.

For a disabled person in the role of a passenger or even driver, the process of transferring from the wheelchair to the automobile seat is usually a demanding operation, and very often, these persons are dependent on the help of others. Only physically fit individuals in good condition can manage this demanding operation on their own, but then they either have to use a simple and lightweight folding wheelchair that they can put in the automobile themselves, or they have to leave the wheelchair outside the automobile.

If the person being transported has to remain in the wheelchair during the transport by automobile, they either have to use a ramp or a lift, or additional special single-purpose handling devices must be installed in the automobile. These devices are always quite complicated and therefore expensive and limit the person being transported as well as the user of the automobile and thereby limit the independence of the mobility-disabled person. A solution for transferring a wheelchair using a lifting ramp is described, for example, in the document FR2726182 A1. Another solution for transferring a wheelchair is known from the document WO200640886 A1. According to this document, to transfer the wheelchair, it is necessary to back the wheelchair onto a part of a handling mechanism, then the wheelchair is lifted by an arm of the mechanism and transferred into the automobile, and finally, the wheelchair must be rotated so that it faces in the direction of the travel. However, this mechanism is quite demanding in terms of structure and space, both the space required for the mechanism itself and the space required around the mechanism to allow the wheelchair to move freely during the transfer to avoid contact between the wheelchair or user and the automobile, which could lead to injury to the user or damage to the wheelchair or automobile. In addition, the precise backing of the wheelchair onto the arm of the mechanism may not be easy or even possible for persons with reduced mobility.

A further mechanism for transferring a wheelchair is described in the document WO201239169A1. This mechanism requires the wheelchair to be positioned parallel to the direction of the travel next to the automobile, which can be relatively difficult with the door of the automobile open. Here, the wheelchair is only moved in the direction of the transverse axis of the automobile and possibly also in the vertical direction, so the open door of the automobile must provide a large amount of free space for the wheelchair to move in and out.

A further solution for transferring a wheelchair into an automobile is described in the document FR2699129 A1. In this solution, the wheelchair must be precisely backed up to a transport arm to which the wheelchair is fixed. Only the seating part is then transferred from the wheelchair, while the wheels must be dismounted before the transfer. During the transfer, the wheelchair then slides along the arm that is fixed in the automobile rotationally and slidably in the vertical direction. In addition to the need to remove the wheels of the wheelchair before the transfer, another disadvantage of this mechanism is its size and structural complexity as well as its very limited range of movement. In addition, the wheelchair must be structurally adapted for handling by this solution.

Further documents describing an assembly for transporting a wheelchair to an automobile are US4664584A and EP0954271. Even these documents leave a room for improvement regarding the range of movement of the assembly and the space it requires in the automobile.

It would therefore be suitable to come up with a solution that would allow for more comfortable transport of the wheelchair to and from the automobile and that would take up relatively little space in the automobile and be usable in as many types of automobiles as possible, both from a technical, ergonomic, and economic point of view.

### Summary of the Invention

The shortcomings of the solutions known from the state of the art are to a certain extent eliminated by an assembly for transporting a wheelchair for transporting persons with disability to and from an automobile. The assembly is defined by the independent claim 1, a method of using the assembly is given in claim 15.

This assembly comprises a support column for attachment to a floor of the automobile, an arm connected to the support column, and a drive mechanism for moving the arm. The arm is provided with an attachment for attaching the wheelchair to the arm. The drive mechanism comprises a lifting guide element with a lifting drive for vertically sliding the arm relative to the support column, a sliding guide element with a sliding drive for horizontally sliding the arm relative to the support column for changing radius of rotation of the wheelchair around an axis of the support column when the wheelchair is attached to the attachment, and a rotating guide element with a rotating drive for rotating the arm around the axis of the support column.

When viewed in direction of axis of the support column, axis of the sliding guide element is offset from the support column. The drive mechanism further comprises a control unit adapted for operating the lifting drive, the sliding drive, and the rotating drive. The drive mechanism comprises a connecting element that is slidably attached on the support column and moves together with the arm when the arm slides vertically, wherein the arm is slidably attached to the connecting element and the sliding drive is adapted for sliding the arm horizontally relative to the connecting element.

The control unit preferably comprises pre-programmed instructions for operating the drives for transferring the wheelchair to and from the automobile, wherein during use of the assembly the user can execute these instructions and thus initiate the transfer of the wheelchair but cannot change them or move the arm along any other than a preset trajectory. Therefore, the control unit preferably contains program instructions specific to the particular structure of the assembly, the automobile, and the wheelchair, which, when executed, ensure the movement of the arm with the attached wheelchair to the automobile and from the automobile without collision. This reduces the risk of injury to the user or damage to the assembly, wheelchair, or automobile by making inappropriate movements of the arm or other parts of the assembly.

The attachment on the arm may comprise a platform for the wheelchair to drive onto. Preferably, the platform comprises an element for firmly securing the wheelchair to the platform, for example a sliding pin that slides into the structure of the wheelchair from below when the wheelchair is driven onto the platform, or the wheelchair may comprise an analogous element for securing to the platform. In this variant, the arm is therefore extended outward before the wheelchair is driven to the automobile. On the other hand, in the variant where the arm is fixed to the structure of the wheelchair by means of the attachment without the need to drive the wheelchair onto the part of the arm, the arm can be extended only after the wheelchair has been driven into place, but it is also possible to drive the wheelchair into the right place after the arm has been extended. For example, the attachment may be implemented as an opening in the frame of the wheelchair or auxiliary frame, as described below, adapted for a metal element (e.g. a pin, mandrel or other technically known connection between two mechanical assemblies) on the arm. This element, or a plurality of such elements, is inserted into said complementary opening, i.e., the attachment on the wheelchair, for mechanical connection of the wheelchair to the assembly. The attachment can be mainly on the back side of the wheelchair, but it is also possible to place it on the side or underneath. Alternatively, the opening can also be placed on the arm and the protruding element on the wheelchair.

Preferably, the support column is adapted for rotational attachment to the floor, particularly preferably also to the ceiling structure of the automobile. For example, the assembly may comprise a base for attachment to the floor in which the support column is housed rotationally. The arm then only slides along two axes relative to the column, which is structurally simpler to implement than also implementing the rotational movement of the arm relative to the column. In addition, rotating the entire column with the arm ensures that the arm cannot hit other parts supported by the column when the arm rotates. The linear guide elements are preferably implemented by means of threaded rods connected to the drives and by means of complementary internal threads, by which the rotation of the threaded rods is converted into a sliding movement. In general, however, other types of linear drives, e.g. hydraulic ones, can also be used.

The support column can be attachable to the floor of the automobile, for example using screws, and can also be attachable to the ceiling of the automobile for better stability or to the window frame of the structure of the body. The length of the column and the length of the lifting guide element define the possible height of the lift of the arm and therefore the height by which the assembly can lift the wheelchair. Preferably, this height is therefore greater than the distance of the door sill of the automobile from the road, but it is also possible to have the assembly designed for use with a wheelchair with the possibility of lifting the wheels or for use with an access ramp. For example, the height of the lift can be at least 300 mm, more preferably at least 350 mm.

The range of rotation of the arm / support column can be e.g. 80-130°, preferably at least 90°. The maximum rotation of the arm α defines an angle at which the wheelchair must approach, in particular in reverse, the arm for transfer to the automobile. This angle is measured from the initial zero rotation of the arm, which corresponds to the direction parallel to the longitudinal axis of the automobile, or the position where the horizontal slide of the arm relative to the support column is parallel to the longitudinal axis. This angle is determined by the structure of the assembly and the automobile and the placement of the assembly in the automobile, for example the position of the support column relative to a B-pillar if the automobile contains one. The choice of this angle can also be influenced by the place where the arm is attached to the wheelchair, i.e., for example, whether the arm is attached to the left or right side of the wheelchair. The arm is preferably straight, with the possible exception of the attachment, i.e. the part of the arm in direct contact with the wheelchair during the transfer. A straight arm allows a greater range of movement than, for example, an "L" arm or an otherwise curved or bent arm.

The size of the horizontal slide of the arm defined by the sliding guide element may be, for example, 20-100 cm, preferably e.g. 20-60 cm. Preferably, the size of this slide is at least half the length of the arm. The horizontal slide allows to change the radius of rotation of the wheelchair around the axis of the support column and thus to carry out the transfer of the wheelchair in a smaller space. In particular, it makes it possible to avoid the door or steering wheel when rotating the wheelchair. Since the whole arm slides, not just, for example, the wheelchair along the arm, the arm itself does not get in the way when rotating, sliding, or during the travel. By using the column as the main structural element of the assembly, for example instead of a larger frame, more space is provided for the movement of the wheelchair, as the wheelchair can extend past the column when viewed from the side. The support column, even with a possible base and with the lifting guide element and lifting drive may occupy for example less than 1500 cm2 when viewed from above, e.g. less than 1000 cm2, preferably less than 700 cm2.

The assembly of the invention can be used in a wide range of automobiles due to its relatively small dimensions and large range of movement. For example, the possibility of a considerable vertical lift of the wheelchair allows the assembly to be used in automobiles almost regardless of the height of the floor/sill from the ground. In addition, the use of column and vertical drives provides a solution that is structurally relatively simple, making installation and servicing easier.

When viewed in the axis of the support column, the sliding guide element preferably passes next to the support column. When viewed in the direction of the slide defined by the sliding guide element, or in projection in a plane perpendicular to that direction, then preferably the arm, or at least the arm excluding the attachment, does not intersect the support column such that the column does not limit the range of the slide of the arm. The axis of the sliding guide elements, e.g. the axis of a threaded rod or the axis of a rail, is then offset from the support column enough such that the arm can slide next to the support column.

The drive mechanism preferably comprises a connecting element slidably attached on the support column, wherein the arm is slidably attached to the connecting element and the sliding drive is adapted to slide the arm relative to the connecting element. Thus, when the arm slides vertically, the connecting element moves together with the arm and when the arm slides horizontally, the arm moves relative to the connecting element. Thus, both the lifting guide element and the sliding guide element can pass through the connecting element.

The control unit may be adapted for operating at least two drives from the lifting drive, the sliding drive, and the rotating drive simultaneously. During the transfer of the wheelchair, the movements performed by these drives do not take place exclusively in sequence but at least partially simultaneously. Thus, for example, the wheelchair may be simultaneously lifted and brought closer to the support column and/or brought closer to the support column and rotated around the axis of the support column. This can reduce the time required for transfer while maintaining a safely low speed of the movement.

The attaching of the arm to the wheelchair can also be done by the user driving the wheelchair to a position suitable for attachment and then the control unit, for example based on data from a sensor of the position of the wheelchair, operates the drives to rotate and/or move the arm by the attachment to the complementary place on the wheelchair. Thus, the user does not need to drive the wheelchair directly to an exact place for connecting to the arm but simply drives the wheelchair to an approximate position to the open door and the attachment is provided automatically by the assembly. The state of this complementary place on the wheelchair where the arm is to be attached is then preferably monitored by at least one sensor to check the correct (i.e. safe) mechanical connection and/or disconnection of the wheelchair to the support arm.

For transporting the wheelchair into the automobile after the wheelchair has been attached to the attachment, the control unit can be adapted to operate the drives for lifting the wheelchair, bringing the wheelchair closer to the support column, rotating the wheelchair around the column, moving the wheelchair away from the support column, and placing the wheelchair on the floor of the automobile. As noted above, these movements may occur in sequence, but they may also occur simultaneously, at least in part. Bringing the wheelchair closer to the support column during or after its rotation reduces the space requirements for the transfer of the wheelchair, i.e., for example, preventing the front end of the wheelchair or the user's foot from colliding with the door, the edge of the opening for the door, or the steering wheel.

The assembly may further comprise at least one sensor for detecting at least one body part of the user seated on the wheelchair during the transport, wherein the control unit is data-connected with the at least one sensor and is adapted to stop the drives upon detection of the user's body part in a predetermined space. This space can be, for example, the space above the headrest of the wheelchair, where if the head extends beyond the headrest there is a risk of injury against the ceiling of the automobile, or the space in front of the footrest where there is a risk of injury to the foot against the door or the edge of the opening of the door. The sensors can be, for example, optical, infrared, ultrasonic, etc. The control unit is then adapted to detect that a part of the user or the wheelchair would come into contact with a part of the automobile during the transfer and stop the transfer or prevent it from initiating. Similarly, alternatively or additionally, the presence of persons in the area of movement of the parts of the assembly may be detected, for example next to the automobile before the arm is extended or near the support column inside the automobile.

A part of the assembly may be a communication unit for communicative connection to the operating unit of the wheelchair. This communication can be wired, initiated after the wheelchair is attached, or wireless. For example, the control unit of the assembly can then control certain functions of the wheelchair, such as tilting the backrest or lifting the wheels, identify that the wheelchair needs to be recharged, etc. Conversely, it is also possible to control the assembly using operating elements on the wheelchair. The assembly preferably also allows recharging the wheelchair from the automobile. The communication unit can be a part of the control unit or it can be a separate part. The data/communicative connection between the wheelchair and the assembly/automobile may include transmission of diagnostic data about the user acquired by the wheelchair (e.g. data from pressure sensors for monitoring pressure sores), transmission of data about the position, etc. These data may then be sent further outside the automobile, for example to predefined recipients, e.g. a doctor.

Furthermore, the assembly may comprise a mechanism for blocking the movement of the door of the automobile. This mechanism secures the door against unwanted closing when the wheelchair is being transferred. The securing of the door can be carried out automatically during each transfer to and from the automobile.

When viewed from above, in each position of the arm, the assembly is preferably arranged for placement of the arm, the support column, and the drive mechanism with their entire volume on one side relative to the longitudinal axis of the automobile. Thus, after the assembly is installed in the automobile, these parts do not extend into the other half of the automobile in any position in which they are placed during the transfers. The mechanism therefore does not extend past the longitudinal axis, so for example there is no risk of injury to the passenger in the next seat or two assemblies can be placed side by side. However, variants of the assembly are also possible where the arm extends beyond the longitudinal axis in some positions during the slide but preferably not in the position where the wheelchair is seated in the automobile.

The assembly may further comprise an auxiliary frame that is adapted to be attached to the wheelchair for transporting persons with disability. The attachment can be, for example, by welding or screwing. The auxiliary frame can be adapted in shape and size to the specific type of wheelchair. The attachment on the arm is then preferably adapted for attachment to the auxiliary frame. The auxiliary frame is therefore an element allowing the assembly of the invention to handle any wheelchair which may not have been adapted for this purpose during the manufacture. Electronic connectors for powering the wheelchair or its communicative connection with the assembly can also be a part of the auxiliary frame. A single connector, or a plurality of separate connectors, can be used for data/communicative and power connection both when connected by the auxiliary frame and when the arm is connected directly to the wheelchair. For example, the auxiliary frame can be a U-shaped profile that surrounds a part of the frame of the given wheelchair from the back, it can be a plate attached to the backrest of the wheelchair from the back or to the seat from below, etc.

The assembly may comprise a wheelchair for transporting persons with disability comprising a chassis provided with a pair of front wheels and a pair of back wheels, a seat, a backrest, a footrest, a power source, and a travel drive. It can also include a control unit for operating the drives, controlling the recharging, etc. One pair of wheels may be driven, but it may be more preferable in terms of driving properties if all four wheels are driven. The backrest comprises a frame of the backrest and the seat comprises a frame of the seat movably connected to the frame of the backrest, to the footrest, and to the chassis. These movable connections can be rotational, sliding, or rotational and sliding. The front pair of wheels is held by the chassis at a constant distance from the back pair of wheels, or the axes of rotation of these wheels defined by their axles are held at a constant distance. The frame of the seat is connected to the chassis by a positioning arm that is rotationally connected to the frame of the seat at its upper end and rotationally connected to the chassis at its lower end. The rotational connection of the positioning arm to the chassis preferably does not slide relative to the chassis or the wheels when the positioning arm moves relative to the chassis. The positioning arm is therefore not a part of a scissor mechanism. However, it changes its inclination relative to the chassis and the frame of the seat during movement, i.e. it basically tilts relative to the chassis.

In addition, the rotational connection of the positioning arm to the chassis is located at the front third, more preferably the front quarter, of the length of the chassis. Furthermore, the wheelchair comprises a positioning linear drive for tilting the positioning arm relative to the chassis and this drive is connected to the chassis at one end and connected to the positioning arm at the other end. These connections are preferably rotational. The connection between the positioning linear drive and the chassis is located closer to the back wheels than the rotational connection between the positioning arm and the chassis. This ensures that the positioning arm tilts backward, i.e. the center of gravity of the wheelchair moves backward when the arm tilts and the seat moves toward the chassis. Preferably, however, the center of gravity in each position, when the wheelchair is placed on a horizontal surface, remains between the front wheels and the back wheels or their axles / axes of rotation when viewed from above.

The travel drive is preferably an electric motor, wherein the power source is a battery. The driven wheels are preferably the back wheels or all four wheels. When using omnidirectional wheels, it may be more convenient or structurally easier to drive the second pair of wheels. The diameter of the wheels is preferably at least 20 cm, more preferably at least 25 cm. The wheelbase of the wheelchair may be less than or equal to 100 cm, more preferably less than or equal to 80 cm, even more preferably less than 70 cm, for example approximately 60 cm. The small wheelbase of the wheelchair is particularly advantageous due to the possibility of transferring the wheelchair into the automobile. This, preferably in combination with the omnidirectional wheels, as will be described below, ensures good driving properties of the wheelchair - stability when crossing obstacles, small radius of rotation of the wheelchair, etc. The weight of the wheelchair is preferably below 100 kg, more preferably below 90 kg, even more preferably below 80 kg, for example around 75 kg. The dynamic stability (nominal inclination) of the wheelchair is preferably at least 6°, the static stability at least 9°. The height of the obstacle that the wheelchair can cross or climb over is preferably at least 50 mm. Preferably, the wheelchair of the invention is a class B wheelchair according to the harmonized standard EN 12184:2014.

Preferably, the wheelchair comprises a framework made of metal profiles, where the chassis, the positioning arm, the seat, the backrest, the footrest, and the armrest or the headrest are partly formed from these profiles. The armrests can be preferably positioned by rotating and sliding the rotational connection vertically so that the position of the armrest can be adjusted according to the needs of the specific user. Furthermore, the wheelchair comprises electronic or drive components such as drives, source, control electronics, operating and display elements, etc. The framework can then be provided with covers and padding. The structure of the framework of the profiles allows to create a relatively lightweight wheelchair, e.g. with a maximum weight of 100 kg, and allows to achieve a high degree of compactness and simultaneously mobility and adjustability of the wheelchair. The use of the covers then protects the framework, protects the user, and makes it possible to create a wheelchair that is valuable not only in terms of function but also design.

The structure of the wheelchair of the invention allows the seating part of the wheelchair to be lowered enough such that the user can, for example, pick up an object from the ground by themselves or help a child to dress. Preferably, it is also possible to raise the seating part above the level intended for standard travel to improve the user's spatial reach. The positioning arm tilts onto the chassis when the seat slides down, it may be possible for the seat to even sit on the chassis or for the positioning arm to lean against the chassis in the lowest position of the wheelchair. This allows the seat to be brought considerably closer to the ground. In addition, the lowering of the seating part, compared to the standard travel position or the height of conventional wheelchairs from the state of the art, allows the wheelchair to be driven e.g. under a table or sink for better access and user comfort when dining, working, etc.

In addition, the positioning of the parts of the wheelchair, especially the footrest and/or backrest, allows the seated user to stretch, relieve long-term strained areas, use for rehabilitation exercises, etc. In addition, when using the control unit to operate the drives to move these and possibly other parts of the wheelchair, the control unit can be adapted to perform movements/readjustments of the parts of the wheelchair automatically, periodically, or in certain cycles. The adjustment of the individual parts, in particular the adjustment of the height of the seat above the chassis, the inclination of the seating area, the footrest, the inclination of the backrest, the position of the armrest, and the position of the headrest, can be individual and can also be linked together. For example, when the seat is lowered, the seating area can be continuously tilted backward, the footrest can be lifted, and the backrest tilted. In addition, the positioning of the parts of the wheelchair, in particular the lowering of the seat to the chassis and the tilting of the backrest to the seat, possibly together with the tilting of the footrest, also makes it easier to store the wheelchair when not in use, place it in the trunk of the automobile, etc. The continuous adjustability of the height of the wheelchair can preferably be used to raise the seat above the level of its use for driving. This is preferable when the user needs to reach higher placed objects or needs to see above a higher placed obstacle (frame of a window, etc.).

Thanks to the use of the positioning arm, the seating part of the wheelchair can be height-positioned in a relatively wide range, e.g. at least 25 cm or at least 30 cm or at least 35 cm. Thanks to the attachment of the arm to the chassis at the front and its tilting back, when the seating part, in particular the seat and all parts attached to it, moves down, this part also moves backward such that the center of gravity of the wheelchair also moves backward. This increases stability, for example when driving downhill or handling objects in a forward bend. In addition, the movement of the seating part backward when lowering the wheelchair helps when using the wheelchair as an automobile seat, especially when the user of the wheelchair is to drive the automobile. For placement in the automobile, it is usually advisable or necessary to adjust the wheelchair to a lowered position and moving the seating part backward adjusts the seat into a more suitable position relative to the steering wheel. In addition, the very possibility of height-adjustment of the seat relative to the chassis with wheels significantly facilitates the transfer of the wheelchair to the automobile, where during the preferred attachment of the transfer mechanism behind the seating part of the wheelchair it is not necessary to lift the entire wheelchair to a large extent, because the collision of the wheels with the sill of the vehicle is prevented by lifting the chassis relative to the seat.

The wheelchair of the invention is relatively lightweight and compact thanks to its structure, so it is easy to handle and can be loaded into a wide range of types of automobiles. In addition, thanks to the range of positioning of the wheelchair, particularly the height-adjustability of the seat, the operational range of the wheelchair is also greatly expanded, from lifting objects from the ground to working on higher positioned work surfaces. This also reduces the user's dependence on assistance. The maneuverability, driving safety, or design of the wheelchair is not compromised in any way. When used with said wheelchair, the assembly of the invention is particularly preferable, because by lifting the chassis relative to the seating part, the required amount of lift of the wheelchair by the arm for the transfer is reduced. In addition, the large compactness of the wheelchair allows the wheelchair to be seated relatively low in the automobile, which is more practical for driving in most passenger automobiles. The relatively lightweight structure of this wheelchair also facilitates the transfer and reduces the stress on the assembly. In addition, the wide range in which the wheelchair and its parts can be positioned allows the wheelchair to be adjusted to a position suitable for driving.

The positioning arm preferably comprises a parallelogram comprising two profiles of the arm mutually offset in the longitudinal direction of the wheelchair, wherein each profile is rotationally connected to the chassis and to the frame of the seat. There are then two rotational connections between the positioning arm and the chassis, preferably both on the front quarter of the chassis, and together they ensure the tilting of the arm. However, it is possible to place only one of these connections on the front quarter with the other further back, but for example still in the front third of the length of the chassis. The length of the chassis may be measured as the length of the structure to which the wheels are attached but may also be measured as the distance between the most distant points of the front and back wheels when viewed from the side. The use of a parallelogram allows the seat to maintain an approximately horizontal orientation during the tilting (rotation of both profiles of the arm). The positioning linear drive can be connected e.g. to the profile of the arm located more in the front. In the most lowered position of the seat, the profiles of the arm may abut against each other and may be essentially parallel to the chassis in this position, for example, they may be at an angle of less than or equal to 20° to it. This allows the seat to be lowered considerably, bringing it closer to the ground, while maintaining sufficient firmness of the structure.

The rotational connection between the positioning arm and the chassis can be located between the front wheels of the wheelchair. In case a parallelogram is used, both these connections are preferably located between the front wheels. Alternatively, the parallelogram may be replaced by another suitable mechanism, e.g. a one-piece arm provided with a suitable rotational connection at each end.

The seat may be adjustable by the positioning linear drive to two extreme positions, wherein the lowest positioned point of the seating area of the seat is in the first, folded, position a maximum of 35 cm higher, more preferably a maximum of 30 cm higher, more preferably a maximum of 25 cm higher than the lowest positioned point of the wheels (i.e. higher than the ground when the wheelchair is placed on level ground) and in the second, standard, position it is at least 45 cm higher, preferably at least 50 or at least 55 cm higher than the lowest positioned point of the wheels. The positioning range between these two positions is preferably at least 15 cm or at least 20 cm. This ensures sufficient height-adjustability of the seat, both for standard travel, handling objects on workbenches, etc., and for handling objects on the ground, etc. In both of these positions, the approximately horizontal orientation of the seating area is still ensured such that it is still possible to sit on the wheelchair. In particular, for example, the inclination of the seating area does not exceed 30°, more preferably 25° or 20° even in the lowered position.

The wheels of at least one pair of wheels are preferably omnidirectional wheels, that is e.g. wheels having additional rolling elements on the circumference, with axes of rotation perpendicular to the axis of rotation of the wheel. For example, at least the front wheels can be omnidirectional. The omnidirectional wheels increase the maneuverability of the wheelchair, especially when turning, which can, for example, make it easier to pass through narrow spaces. It may also be possible to rotate the wheelchair in place, i.e. around the axis that intersects the wheelchair when viewed from above.

The frame of the seat may comprise a top piece and a bottom piece that are movably connected to each other, wherein the bottom piece is connected to the positioning arm, the top piece carries the seating area, and the top piece is tiltable relative to the bottom piece. This tilting can be, for example, in the range of a maximum of ±15° or ±10°. The footrest, backrest etc. can be attached to the top or bottom piece. Thanks to the tilting of the top piece relative to the bottom one, it is possible to tilt the seating area of the seat relative to the chassis. For example, user comfort can be increased when the backrest is tilted if simultaneously the seating area is tilted. This function can be further utilized when driving downhill or uphill to increase the stability of the wheelchair and improve user comfort. When sitting in the wheelchair for a longer period of time, tilting the seating area can make it possible to change the places most stressed by sitting and thus to prevent pressure sores. The top and bottom pieces may for example be rotationally connected by a pin, or such a pin may also slide relative to one of the pieces. The tilting of the pieces can thus be a rotational movement or a combination of rotational movement and slide.

The wheelchair for transporting persons with disability may further comprise at least one of the following drives or any combination thereof: a drive for rotating and/or extending the footrest, a drive for rotating the backrest relative to the seat, a drive for tilting the top piece of the frame of the seat, a drive for rotating the armrest, and a drive for moving the headrest. Particularly preferable is the use of a drive for moving the backrest, for example together with a drive for moving the footrest and for tilting the seating area, i.e. the top piece of the frame of the seat. The drives can be powered from the same source as the travel drive of the wheelchair. They can be operated directly by the user via operating elements and/or can be connected to the control unit that controls the drives automatically or according to the user's instructions.

The wheelchair for transporting persons with disability may comprise an attachment for attaching the arm of the assembly for transporting the wheelchair to an automobile. The attachment may be, for example, an opening for inserting the end of the arm of the assembly, may comprise a lock for locking the arm of the assembly, etc. Preferably the attachment is on the seat, but it may also be located, for example, on the chassis or backrest. The wheelchair may further be adapted for communication with such an assembly, for example to adjust the positions of the parts of the wheelchair during the transfer of the wheelchair to the automobile. For example, the wheelchair comprises one or more connectors for data or power connection to the assembly and/or automobile. The control unit of the wheelchair can therefore establish a connection to the electronics of the automobile or the assembly for transporting the wheelchair via this connector, furthermore, charging of the wheelchair from the automobile can also be carried out via the connectors, etc.

The wheelchair may further comprise an auxiliary frame that is attached to the wheelchair and comprises an attachment for attaching the arm of the assembly for transporting the wheelchair to the automobile. The attachment can be, for example, by welding or screwing. The auxiliary frame can be adapted in shape and size to the specific type of wheelchair. Thus, the auxiliary frame is an element enabling any wheelchair of the invention to be adapted for transport by means of the given assembly for transporting the wheelchair. Electronic connectors for powering the wheelchair or its communicative connection with the assembly can also be a part of the auxiliary frame. A single connector, or a plurality of separate connectors, can be used for both data/communicative and power connection, both when connected by the auxiliary frame and when the arm is directly connected to the wheelchair. For example, the auxiliary frame can be a U-shaped profile that surrounds a part of the frame of the given wheelchair from the back, it can be a plate attached to the backrest of the wheelchair from the back or to the seat from below, etc.

The movement of the backrest relative to the seat and/or the footrest relative to the seat and/or the top piece of the frame of the seat relative to the bottom piece of the frame of the seat, preferably the movement of all three of these movements simultaneously, may be electronically or mechanically linked to the movement of the positioning linear drive. For example, the mechanical link may be implemented by means of rods, e.g. connecting the top piece of the frame of the seat to the backrest and the top piece of the frame of the seat to the positioning column. Electronic link can be implemented by the control unit operating the drives. The above movements can be linked with the height-adjustment of the seat relative to the chassis. Especially in the case of electronic link, this link can be selective, i.e. individual execution of these movements can be enabled according to the user's requirements.

A control unit adapted for operating the drives of the wheelchair as well as user operating elements data connected to the control unit can therefore be a part of the wheelchair. These elements can be, for example, a joystick, buttons, touchscreen, voice assistant, etc. The control unit may control all or some of the drives, for example it may control the positioning of individual parts, but not the travel drive, which may be controlled directly by user operating elements.

The shortcomings of the known solutions are further to a certain extent eliminated by a passenger automobile that comprises the assembly for transporting a wheelchair for transporting persons with disability of the present invention, wherein the support column is attached to the floor of the automobile. The assembly is then preferably powered from the automobile and can also be communicatively connected to its control unit. The object of the invention can also be an assembly for transporting persons comprising a wheelchair for transporting persons with disability and an assembly for transporting the wheelchair for transporting persons with disability to and from the automobile of the invention. This wheelchair is then adapted for transport by the assembly, in particular for attaching to an attachment.

An automobile with a sliding door near the place where the assembly is to be placed may be more preferable than one with a rotational hinged door. Opening the sliding door can be fully automatic, which is more practical for the wheelchair user, and the sliding door requires less space next to the automobile when opening. In addition, the risk of hitting the door during the transfer is largely eliminated. Particularly preferable is an automobile containing a combination of automatically opening sliding door with the assembly of the invention. It can be preferable to use an electric automobile, either fully electric or hybrid. The battery in such an automobile has a larger capacity and can therefore be used more advantageously to recharge the wheelchair and/or power the assembly. The assembly can also be used with manually driven wheelchairs, but the use for electrically driven wheelchairs is particularly advantageous and the possibility of recharging the wheelchair from the automobile through the assembly of the invention is then particularly advantageous.

The shortcomings of the solutions from the state of the art are to a certain extent eliminated by a method for transporting the wheelchair for transporting persons with disability to the automobile in which:
- The door of the automobile is opened.
- The wheelchair is driven to the open door, wherein the back side of the wheelchair faces the automobile.
- From the open door, the arm attached slidably in a vertical and horizontal direction to the support column fixed to the floor of the automobile and rotating around the axis of this column is extended, wherein the arm is connected to the wheelchair by means of the attachment.
- The wheelchair is transferred by the arm into the automobile, wherein the transfer comprises at least lifting the wheelchair, transferring the wheelchair closer to the support column, and rotating the wheelchair. The movements of the wheelchair or the arm for transferring the wheelchair are preferably predefined in the control unit.

Particularly preferably, a step of lowering the wheelchair may follow, for example by positioning it on the floor of the automobile with the wheels, and/or a step of anchoring the wheelchair in the automobile, e.g. by firmly connecting the wheelchair to the floor of the automobile. The driving of the wheelchair and the extension of the arm may occur in the mentioned order as described above or in reversed order, taking into account particularly the implementation of the attachment. Thus, if the attachment comprises a platform the wheelchair to drive onto, the arm is extended and the platform is placed on the ground before the wheelchair is driven to the automobile. The wheelchair is then preferably firmly secured to the platform such that it cannot be driven off the platform during or after the transfer to the automobile. If the wheelchair is communicatively connectable to the automobile or assembly, the transfer of the wheelchair may comprise blocking the travel drive of the wheelchair and unblocking it after the wheelchair has been transferred out of the automobile.

This method may be implemented by the assembly of the invention and may comprise additional steps corresponding to additional parts or functions of the assembly. For example, the method may comprise a step of sensing the position of at least one part of the user's body during the transfer and preventing the user from colliding with the automobile or the assembly during the transfer based on data from the sensors. Similarly, the position of the parts of the wheelchair can be sensed to prevent the wheelchair from colliding with the automobile or the assembly. The method may comprise a step of blocking the door of the automobile before the transfer is initiated. It may comprise a step of adjusting the position of the wheelchair relative to the steering wheel or other part of the interior after the wheelchair has been transferred into the automobile. It may comprise a step of anchoring the wheelchair to the floor after the transfer is finished. It may comprise a step of closing the door.

### Description of Drawings

A summary of the invention is further clarified using exemplary embodiments thereof, which are described with reference to the accompanying drawings, in which:
- Fig. 1: schematically shows a lateral view of an automobile provided with an assembly of an embodiment of the present invention during the transfer of the wheelchair inside, wherein the assembly is placed in place of the driver's seat such that the user of the wheelchair can drive the automobile after the transfer inside, wherein the wheelchair is partially lifted such that it does not touch the ground with its wheels, but the wheels are still lower than the door sill of the automobile.

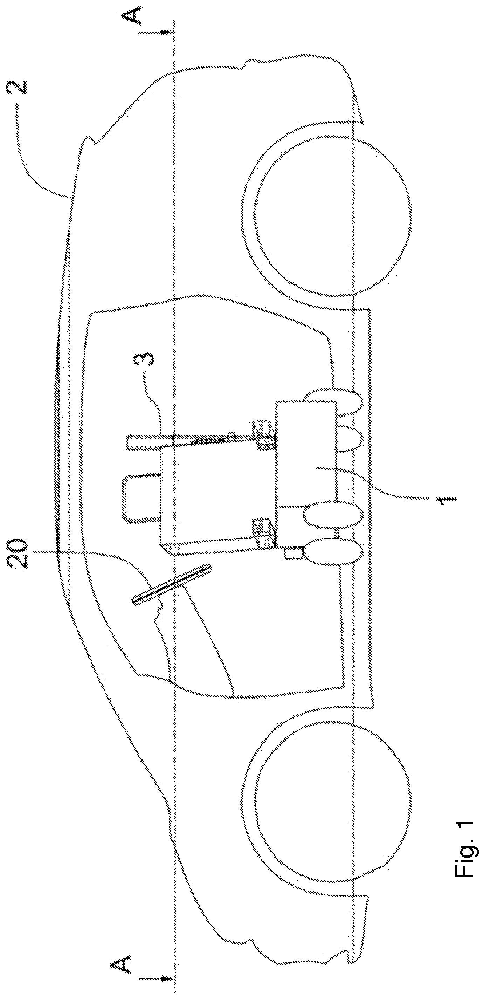

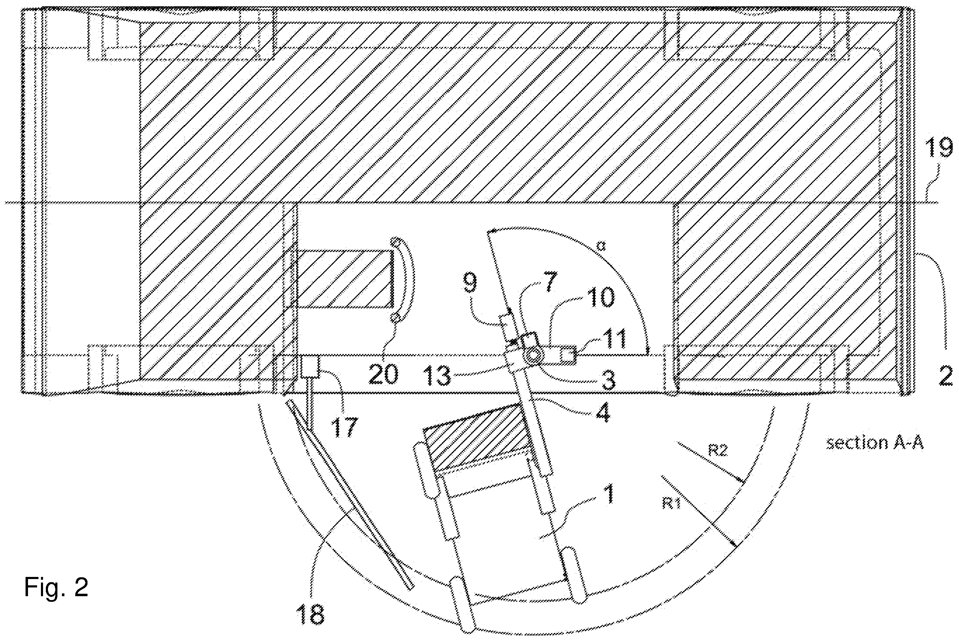

- Fig. 2: is a schematic view from above of an automobile with the assembly in the position shown of fig. 1, wherein hatching indicates a part of the plan of the vehicle which neither the assembly nor the wheelchair extend into during the transfer of the wheelchair, and wherein the radii of rotation of both front wheels of the wheelchair during the rotation of the wheelchair around the axis of the support column are indicated and it can be seen that rotation of the wheelchair in this position would impact the open door of the automobile, wherein furthermore the figure schematically shows a mechanism for blocking the movement of the door.
- Fig. 3: is a schematic lateral view of the automobile of fig. 1 in a position where the wheelchair is lifted to the maximum such that it can be transferred inside without the wheels hitting the sill of the automobile, wherein the view from above of this position corresponds to the view of fig. 2.
- Fig. 4: is a schematic lateral view of the automobile of. figs. 1 and 3 during horizontal transfer of the wheelchair inside, wherein the wheelchair is partially rotated in the direction of travel.
- Fig. 5: is a schematic view from above of the automobile with the assembly in the position of fig. 4, wherein the wheelchair has been transferred closer to the support column by sliding the arm in the connecting element, compared to the position of fig. 2, so it can be seen that the radii of rotation of both front wheels of the wheelchair during the rotation of the wheelchair around the axis of the support column are smaller than in the position of fig. 2 and there is no risk of the wheels hitting the door of the automobile.
- Fig. 6: is a schematic view from the side of the automobile with the assembly in a position where the wheelchair is transferred in a horizontal plane to the final position for driving the automobile, in which the wheelchair is rotated in the direction of travel and transferred closer to the steering wheel.
- Fig. 7: is a schematic view from above of the automobile with the assembly in the position of fig. 6, wherein the wheelchair is partially located under the steering wheel and it can be seen that the radii of rotation of both front wheels of the wheelchair during the rotation of the wheelchair around the axis of the support column are smaller than in the position of fig. 2 and greater than in the position of fig. 5.
- Fig. 8: is a perspective view of the assembly in an exemplary embodiment of the present invention, wherein the attachment comprises two branches for securing the wheelchair from both lateral sides.
- Fig. 9: is another perspective view of the assembly of fig. 8 in a different position of the rotation of the column with the arm and the slide of the arm relative to the column.
- Fig. 10: is a schematic diagram of the connection of the control unit of the assembly with the sensors, drives, mechanism for blocking the movement of the door, and communication unit wirelessly connected to the remote control and the operating unit of the wheelchair.
- Fig. 11: shows a flow diagram of an exemplary embodiment of the method of the invention.
- Fig. 12: schematically shows a lateral view of the wheelchair for transporting persons with disability in one exemplary embodiment of the present invention, wherein the wheelchair is in a standard travel position.
- Fig. 13: is a schematic view from the side of the wheelchair of fig. 12, wherein the covers of the wheelchair are not shown, so the frames and profiles forming the framework of the wheelchair are visible.
- Fig. 14: schematically shows a perspective view of the wheelchair of figs. 12 and 13, wherein the left armrest is lifted to a vertical position to facilitate getting on the wheelchair and getting off the wheelchair.
- Fig. 15: schematically shows a lateral view of the wheelchair in the lowered position, where the seating area is lower and more to the back and the backrest is tilted backward more than in the standard position of fig. 12.
- Fig. 16: is a schematic view from the side of the wheelchair of fig. 15, wherein the covers of the wheelchair are not shown, so the frames and profiles forming the framework of the wheelchair are visible, and wherein the footrest is more extended.
- Fig. 17: is a detailed schematic view from the side of the chassis and the positioning arm of the wheelchair without the covers in the standard position of the wheelchair.
- Fig. 18: is the view of fig. 17 in the lowered position of the wheelchair.
- Fig. 19: is the view of fig. 12, wherein the headrest is extended upward.
- Fig. 20: is a schematic detailed view from the back of the headrest and its attachment to the frame of the backrest.
- Fig. 21: is a detailed schematic view from the side of the armrest with a display unit and an operating element for driving the wheelchair.
- Fig. 22: is the view of fig. 12, wherein the footrest is extended and the backrest with the armrest are tilted backward.

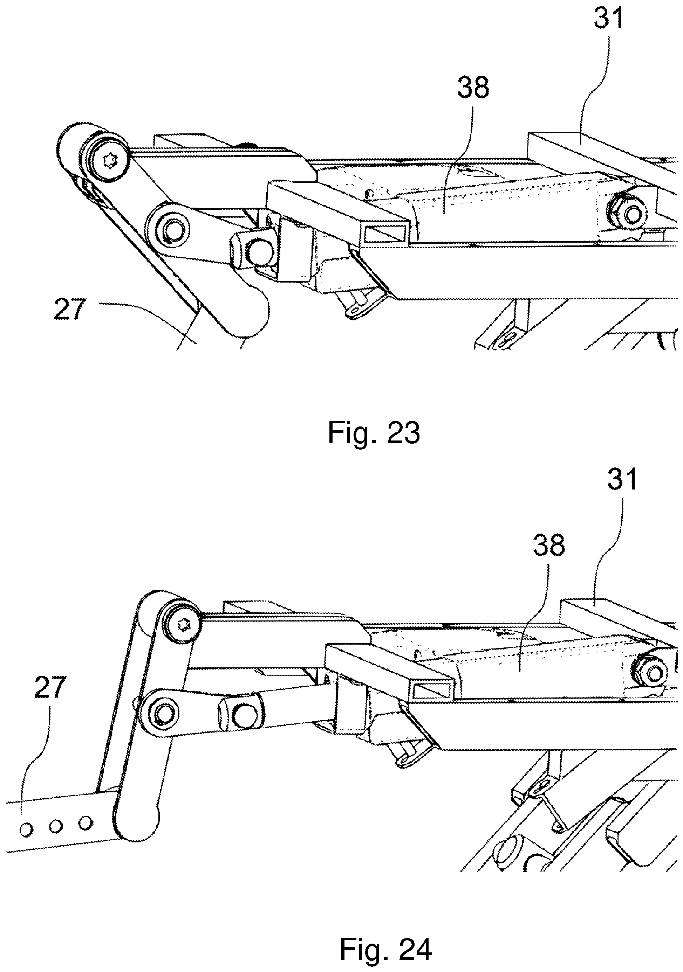

- Fig. 23: is a detailed view of the drive for rotating and/or extending the footrest and of the rods that connect this drive to the footrest.
- Fig. 24: is the view of fig. 23, wherein the footrest is more extended than in fig. 23.
- Fig. 25: is the view of fig. 12, wherein the seating area of the seat is tilted backward and the backrest with the armrest are also tilted backward.
- Fig. 26: is the view of fig. 25, wherein the covers of the wheelchair are not shown, so the frames and profiles forming the framework of the wheelchair are visible.
- Fig. 27: is a detailed schematic view of the mechanism for connecting the top piece and the bottom piece of the frame of the seat, which allows the seating area of the seat to be tilted.
- Fig. 28: is the view of fig. 27, wherein the top piece is tilted backward.
- Fig. 29: is the view of fig. 12, wherein the left armrest is rotated to a vertical position.
- Fig. 30: is a schematic diagram of the connection of the electronic components of the wheelchair to the control unit and the data connection of the control unit to the user device and to the control unit of the assembly for transferring the wheelchair to and from the automobile.
- Fig. 31: is a schematic perspective view of an alternative embodiment of the assembly of the invention with a platform the wheelchair to drive onto.
- Fig. 32: is a schematic perspective view of the assembly of fig. 31, wherein the wheelchair is positioned on the platform and fixed to it by a securing pin.
- Fig. 33: is a schematic perspective view of the assembly in an embodiment with the platform, wherein the assembly is incorporated into a passenger automobile, wherein the assembly is in a position where the platform is extended from the automobile and positioned on the ground.
- Fig. 34: is a schematic view of the assembly of fig. 33 in the lifted position, where the platform is above the level of the door sill of the automobile.
- Fig. 35: is a schematic view of the assembly of fig. 33 in the lifted and slid position, with the platform above the level of the sill and partially located inside the automobile - in this position the wheelchair is already transferred closer to the support column (axis of rotation) and can be rotated on the arm for complete insertion into the automobile.
- Fig. 36: is a schematic view of the assembly of fig. 33 in the position where the wheelchair is transferred to the automobile and the user can drive the automobile.

### Exemplary Embodiments of the Invention

The invention will be further clarified using exemplary embodiments with reference to the respective drawings.

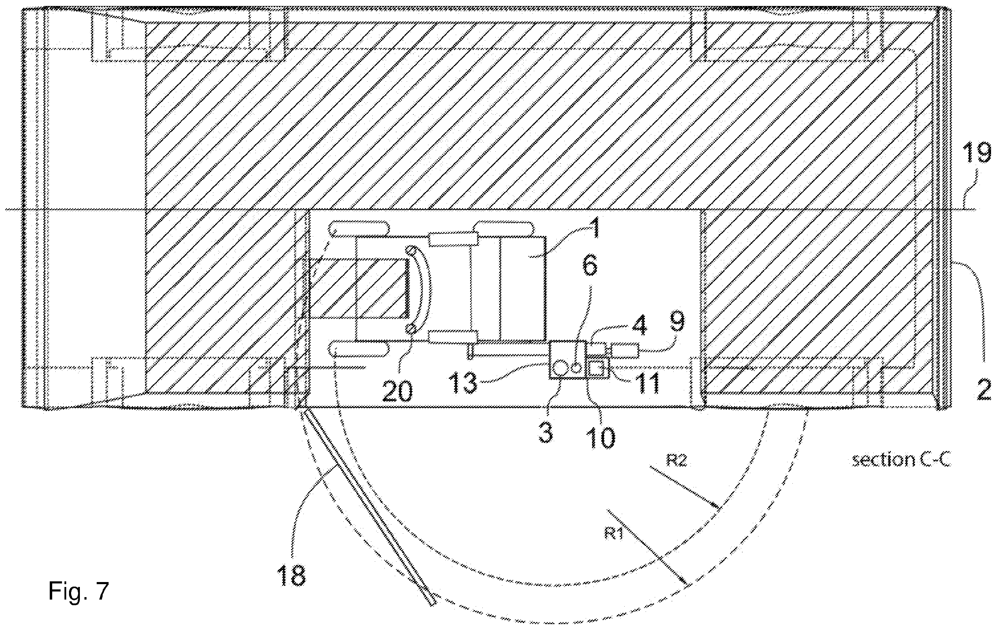

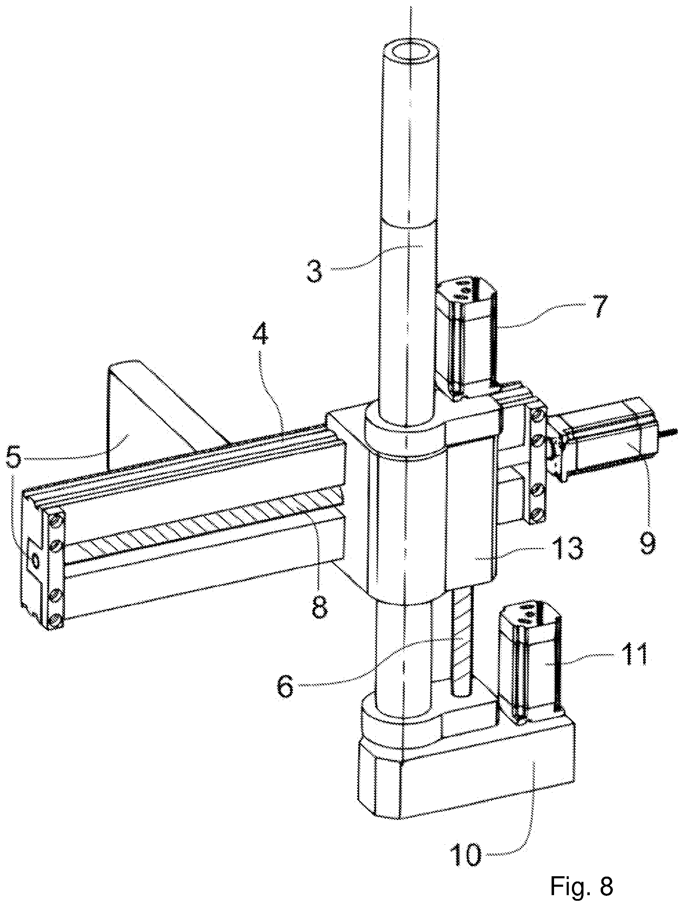

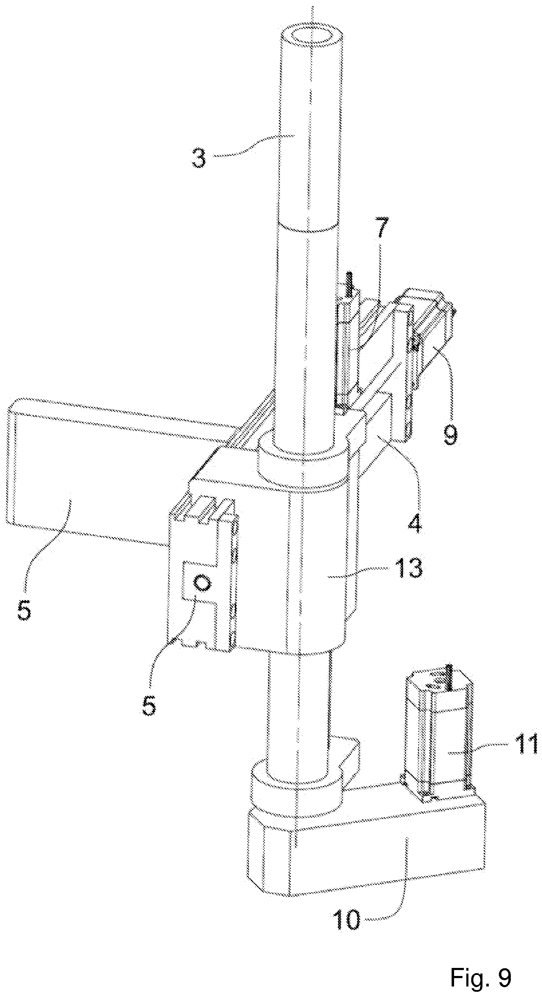

The assembly for transporting a wheelchair 1 for transporting persons with disability to and from an automobile 2 comprises a support column 3, an arm 4 movably attached on the support column 3, and a drive mechanism which provides movement of the arm 4 by rotating around the axis of the support column 3 and by moving in the vertical and horizontal direction after the support column 3 has been attached in the vertical direction to the floor, and optionally additionally even to the ceiling, of the automobile 2. The drive mechanism comprises three drives and three guide elements - two linear and one rotational, for providing said three movements, and further comprises a control unit 12 for controlling the drives. The power supply of the assembly can be provided, for example, by connection to a car battery, or the assembly can be provided with its own power source, for example, recharged from the automobile 2 while driving, but ensuring the transfer of the wheelchair 1 without depending on the (charged) car battery of the automobile 2. A detailed view of an example of the assembly is shown in figs. 8 and 9, the movement of the wheelchair 1 and arm 4 during the transfer can be seen in the views from the side and from above in figs. 1 to 7. The extension of the wheelchair 1 from the automobile 2 (by a combination of rotatory and sliding movements) occurs analogously to the insertion of the wheelchair 1 inside. The order of figs. 1 to 7 corresponds to the transfer inside. The assembly can be used to transfer the wheelchair 1 to the driver's seat but also to the passenger's seat or to the seat for another passenger in the vehicle.

All three movements of the arm 4 relative to the floor of the automobile 2 are preferably independent of each other such that each of them can occur alone or in any combination with the other movements. The slide in the direction of the support column 3 axis, i.e. the up and down movement after the assembly is installed in the automobile 2, is carried out by the movement of the arm 4 relative to the column. Preferably, the arm 4 is connected to the support column 3 via a connecting element 13 which is slidable on the column in the axis of the column. For example, it may be implemented as a sleeve surrounding the support column 3 around its entire circumference and may comprise bearings to ensure smooth movement. Both the lifting guide element 6 and the lifting drive 7 can be built into the column or placed next to it (see fig. 8). For example, a threaded rod can be used rotatably (around the axis of the rod) attached at both ends thereof to the column, floor, or ceiling. In the shown embodiment, it is attached at both ends to the support column 3 by means of rod holders, wherein it can rotate in the holders and is connected from above to the lifting drive 7 - an electric motor. There is then a counter-thread on the connecting element 13 (or alternatively directly on the arm 4 or other intermediate piece of the mechanism) such that the rotation of the threaded rod due to the rotation of the lifting drive 7 is converted into a vertical movement of the connecting element 13 and with it also the arm 4.

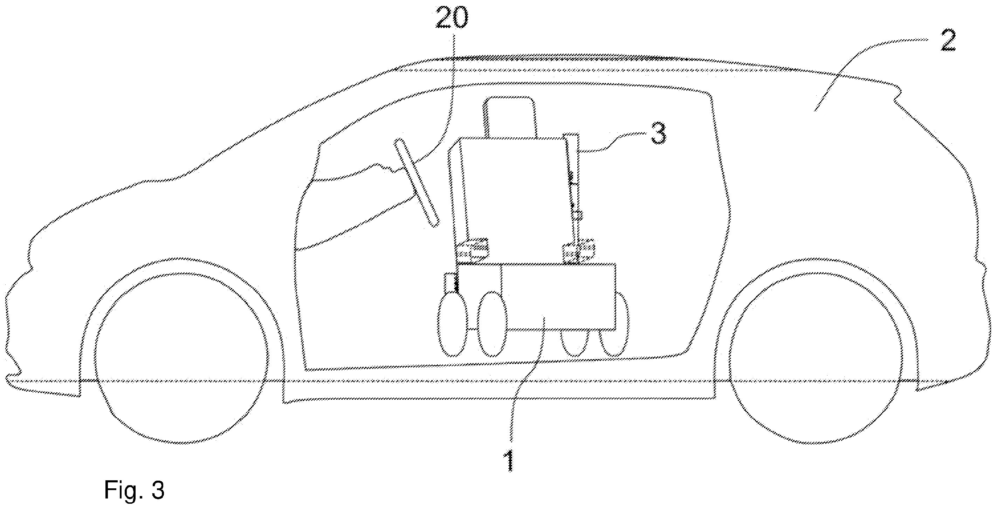

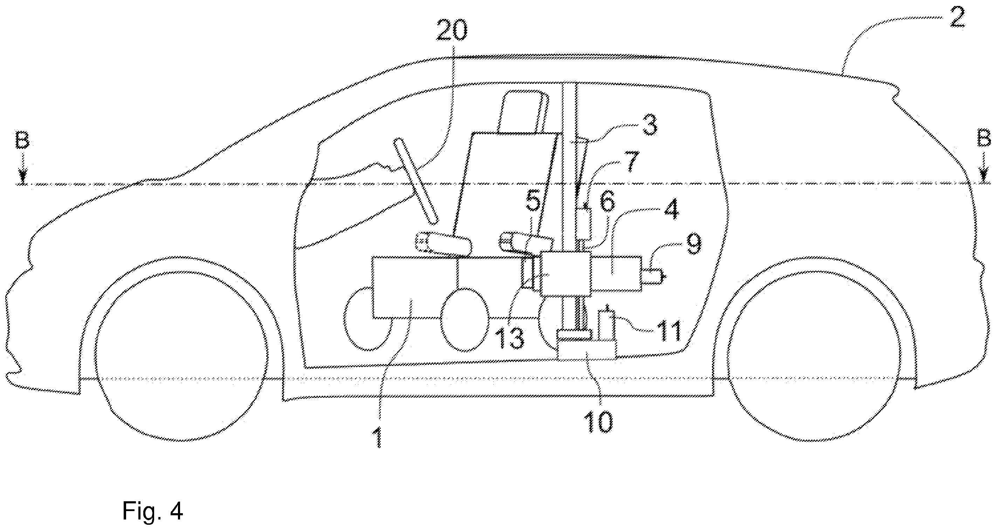

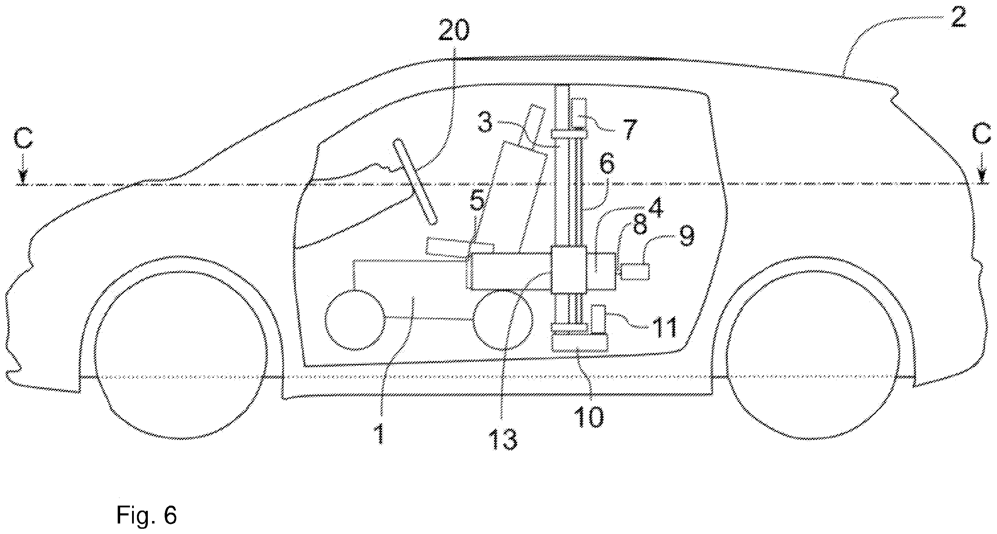

The connecting element 13 is then slidable within the interval defined by the rod holders. The size of this interval can be selected according to the type of the automobile 2 and the required height of the lift of the wheelchair 1. For example, for wheelchairs 1 with adjustable distance of the wheels from the seat or for applications in places with an access ramp at the parking space for the given automobile 2, the lift can be also lower, for example 20-40 cm. In other embodiments, the lift can be also higher, for example 40-70 cm. For example, in figs. 1 and 3, the support column 3 is only fixed to the floor at the bottom, its upper end is free so that a smaller lift is allowed. In fig. 4, the column is also attached to the ceiling, but the upper rod holder is approximately half way up the column, so the height of the lift is limited here as well. Fig. 6 indicates an assembly with a greater height of the lift, where the upper rod holder is near the upper end of the support column 3 attached to the ceiling of the automobile 2.

The rotational movement is preferably carried out by rotating the entire column relative to the floor, such as in the shown embodiment, where the assembly comprises a base serving as a rotating guide element 10 for the column. The rotating drive 11 is located on this base and connected to the column by a gear. Therefore, when rotating, not only the arm 4 but also the connecting element 13, the column, and both linear guide elements and both of their drives rotate. The column may then, except for the bottom and possibly also the upper end, have a non-round cross-section. In some embodiments, the rotating drive 11 may be built into the base or may be located near the ceiling.

The slide perpendicular to the axis of the support column 3, i.e. the horizontal slide, is preferably provided by the slide of the arm 4 relative to the connecting element 13. The connecting element 13 keeps the arm 4 offset from the support column 3 such that the arm 4 can move next the column and therefore the support column 3 does not limit the slide of the arm 4 - this slide is limited by the length of the arm 4 and the sliding guide element 8. The sliding guide element 8 may be a threaded rod with a sliding drive 9, e.g. an electric motor, preferably a stepper motor, located at one end of the rod. The connecting element 13 then comprises a complementary internal thread for sliding the rod in its axis as a result of its rotation. The threaded rod is then rotatably (around its axis) attached to the arm 4, for example on its external surface by means of the rod holders, but preferably it is built thereinto (see e.g. fig. 8). In addition, the sliding movement between the arm 4 and the connecting element 13 may be defined by grooves or rails, as can be seen, for example, in fig. 9.

Instead of threaded rods, a gear rack, a chain or belt driven mechanism, etc., can be used in the assembly for the linear slide. It is also possible to use a pneumatic or hydraulic drive. The parts of the drive mechanism, in particular the threaded rod, bearing, toothing, etc., are preferably located in the cover or built into a different part (such as, for example, inside the support column 3 or arm 4). This prevents possible injuries due to the movable parts of the mechanism, and the mechanism is better protected against contamination.

The attachment of the wheelchair 1 to the arm 4 may be implemented, for example, by inserting the end of the arm 4 into the complementary opening in the frame of the wheelchair 1, by attaching the wheelchair 1 from below, for example in the place under the seat, by an attachment 5 at the end of the arm 4, etc. Preferably, the attaching of the wheelchair 1 is additionally secured, for example, by an electric or electromagnetic securing mechanism, which may be operated, for example, by the control unit 12, to prevent the wheelchair 1 from sliding off the arm 4 spontaneously during the transfer. For example, the arm 4 may comprise a sliding mandrel or pin that thanks to its movement fits into an opening in the wheelchair 1 and thus the arm 4 is firmly and immovably connected to the wheelchair 1. In the embodiment of figs. 8 and 9, the attachment 5 directly at the end of the arm 4 intended to be secured to the lateral side of the wheelchair 1 is supplemented by a second branch of the attachment 5 that holds the wheelchair 1 from behind, thereby significantly reducing the stress on the attachment 5. Alternatively, it is also possible to use an attachment 5 with a second L-shaped branch, which also secures the other lateral side of the wheelchair 1.

The kinematics of the transfer of the wheelchair 1, i.e. the combination and range of movements performed during the transfer and their speed, is preferably automatic 2 without the user being able to change it easily. For example, it can be set by a service technician during the installation of the assembly, wherein the range of movements corresponds to the specific type of automobile 2 and wheelchair 1 to avoid collision during the transfer. The user then, for example, simply initiates the extension of the arm 4 before attaching the wheelchair 1 or its insertion after removing the wheelchair 1 (the movement of the empty arm 4 can be significantly faster than that of the arm 4 with the wheelchair 1 attached) and initiates the transfer of the wheelchair 1. Alternatively, the user can also control the locking of the wheelchair 1 to the arm 4 or its anchoring to the floor. Preferably, the assembly contains a remote control 21 or it is possible to connect it to a user device, for example a phone using a mobile application. The communication can be carried out, for example, via Bluetooth^{®} or Wi-Fi. It is also possible to control the assembly directly from wheelchair 1, for example if it is equipped with a touch screen display on the armrest.

By using three different movements of the arm 4, and therefore of the wheelchair 1, it is possible to combine these movements to create a path of movement of the wheelchair 1 during loading and unloading that is adapted to the specific shape of the door 18, the type of automobile 2, or wheelchair 1 or the height of the user. In particular, it is preferably possible, when transferring the wheelchair 1 inside the automobile 2, after attaching the wheelchair 1, to lift the wheelchair 1 from the ground, to move it (by horizontal movement of the arm 4) closer to the support column 3 and only then to rotate it. The movement closer to the column, which can take place after the wheelchair 1 is lifted but can also take place simultaneously with the lifting, reduces the radius of rotation, especially for the front wheels of the wheelchair 1 or the user's feet, which are usually furthest from the axis of rotation (the axis of the column) and therefore affect the demands on the space requirements for the transfer the most.

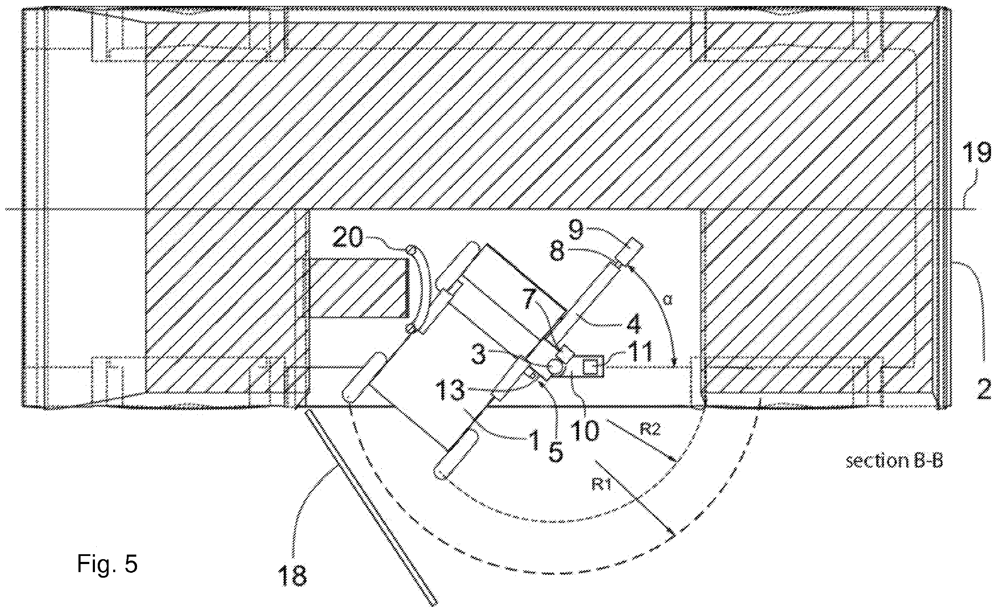

This preferred combination of movements can be seen, for example, when comparing figs. 2 and 5. In fig. 2, the radii of rotation of the front wheels of the wheelchair 1 around the axis of the support column 3 are R1 and R2, where R1 is larger because it corresponds to the wheel on the lateral side further away from the support column 3, maximum. As can be seen from the figure, the opening of the door 18 to the maximum opening angle is insufficient to prevent the wheels of the wheelchair 1 from hitting the door 18. The present invention makes it possible to solve this problem by bringing the wheelchair 1 closer to the axis of rotation, for example the wheelchair 1 can also be partially inserted into the automobile 2 before initiating the rotation. By bringing it closer, both radii are reduced (see fig. 5). For example, R1 may be 1100-1400 mm and R2 may be 900-1300 mm before the wheelchair 1 is brought closer to the column. The angle α describing the rotation of the arm 4 relative to the initial position where it is parallel to the direction of the travel can be, for example, 80-120° in the maximum rotation of the arm 4, in the embodiment of fig. 2 the angle α is equal to 105°. After the wheelchair 1 is brought closer to the support column 3, R1 may be, for example, 700-1100 mm and R2 may be, for example, 500-900 mm. The specific values of these dimensions depend on the specific dimensions of the wheelchair 1 and the parts of the assembly and may also be affected by the preset kinematics of the assembly, for example with respect to the length and maximum opening angle of the door 18 on a specific automobile 2. In addition to the impact on the door 18, the described bringing closer of the wheelchair 1 to the axis of rotation prevents an impact on the steering wheel 20. The rotation of the wheelchair 1 around the axis of the support column 3 may begin only after complete lifting and/or bringing closer but may also occur simultaneously with the bringing closer or simultaneously with both the bringing closer and the lifting. Combining multiple movements can significantly speed up the overall transfer. For example, the transfer may take 30-100 seconds.

As can be seen from the plan views in figs. 2, 5, and 7, the assembly is preferably implemented such that after the installation in the automobile 2 it does not extend past the longitudinal axis 19 of the automobile 2, or rather the arm 4 and the wheelchair 1 are located entirely on one side of the longitudinal axis 19 when viewed from above. It is therefore not necessary to interfere in any way with the structure of the passenger seat or to manipulate it, and it is even possible to place two assemblies side by side in one automobile 2.

The assembly may further include a mechanism 17 for blocking the movement of the door 18 of the automobile 2. It may be for example a telescopic strut that rests against the door 18 after the door 18 is opened to prevent the unwanted closing thereof, for example by a gust of wind, as indicated in fig. 2. It is also possible, for example, to implement this mechanism as a brake in the door 18 hinge or a strut slidable vertically from the door 18 to the ground, etc. The control of the mechanism 17 for blocking the movement of the door 18 may be automatic by means of the control unit 12, for example the door 18 may always be blocked before the transfer is initiated. The actual opening of the door 18 may be implemented fully or partially manually, but it may also be implemented electronically, so the assembly may further include a drive for moving the door 18. For example, the automobile 2 with the assembly may be implemented in such a way that the door 18 must be unlocked and slightly opened manually and then it is possible to start its complete opening with the remote control 21.

The assembly may further include sensors 14 for detecting parts of the user's body. For example, these can be distance or motion sensors 14, a camera with a processor and an image recognition module, a thermal imaging camera, etc. can also be used. The control unit 12 is data connected to these sensors 14 and, based on the data from them, which it can evaluate itself or receive them already processed from another computing or control unit, it stops the drives or prevents them from being initiated if the user could be injured or the automobile 2, the assembly, or the wheelchair 1 could be damaged. For example, the sensor 14 may be used to detect user's head extending beyond the headrest of the wheelchair 1 or it may detect the position of the head relative to the ceiling of the automobile 2 or the upper edge of the opening of the door 18 to prevent head injury during the transfer. Similarly, extension of the user's feet in front of the wheelchair 1 or to the sides, extension of the hands, or placement of the hands outside the armrests or the feet outside the footrest of the wheelchair 1 may be detected. Alternatively or additionally, a potential impact of the user or the wheelchair 1 on the steering wheel 20, the door 18, the door 18 sill, the pillar of the automobile 2, etc., may be detected in this way. Preferably the assembly comprises a plurality of sensors 14 for detecting potential injury to the user or damage to the wheelchair 1 in a plurality of places, in particular in terms of the head extending beyond the wheelchair 1 or the feet under the wheelchair 1 or to the front or side of the wheelchair 1. In general, this detection of the presence of a part of the user's body or a part of the wheelchair 1 in a certain place (relative to the wheelchair 1 and/or the automobile 2) can be performed by a control unit other than the one that controls the drives.

Other sensors 14 included in the assembly may be sensors 14 detecting the exceeding of a certain value of force or power in the drives. Such an exceedance may indicate, for example, an overload of the wheelchair 1 above the level of weight safely transferable by the assembly, or an impact of the wheelchair 1 or the user on a part of the automobile 2 during transfer or a foreign object or body part stuck between the wheelchair 1 or the assembly and the automobile 2, etc. An additional sensor 14 may be used to detect proper attachment of the wheelchair 1 to the arm 4 or to detect obstacles next to the automobile 2 before extending the arm 4.

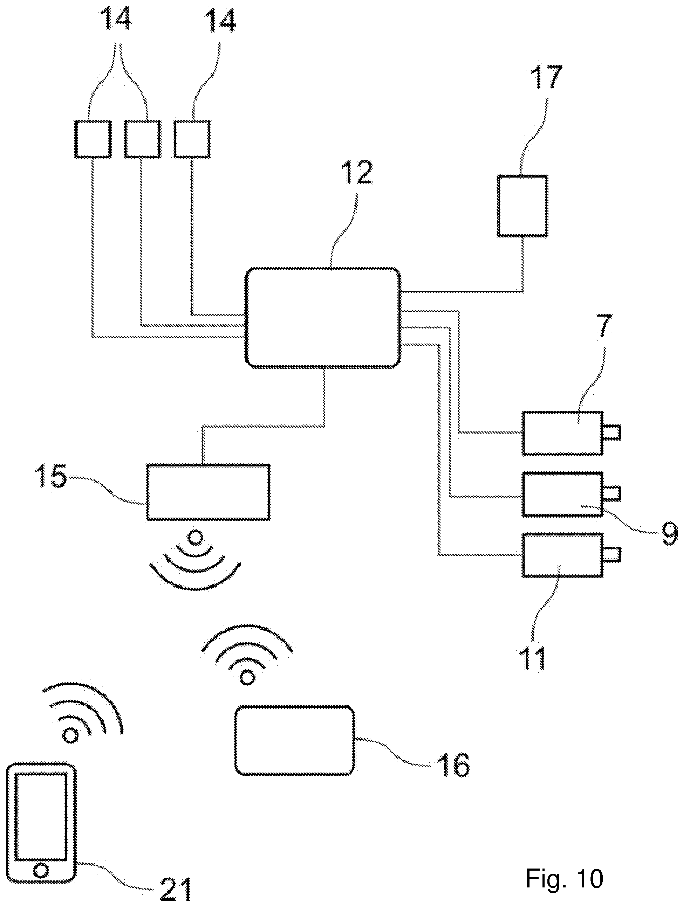

The assembly may further include a communication unit 15 enabling a communicative connection between the control unit 12 of the assembly and the operating unit 16 of the wheelchair u. Preferably, a connector for connecting the wheelchair 1 for recharging it from the automobile 2 is also included. Thus it is for example possible to adjust the wheelchair 1 (position of the wheels, headrest, footrest, etc.) before transfer to avoid collisions by the control unit 12 of the assembly. Conversely, it may be possible to operate the assembly from the wheelchair 1. The connection of the electronic parts of the assembly with the control unit 12 is schematically shown in fig. 10, where the communication unit 15 of the assembly additionally communicates wirelessly with the operating unit 16 of the wheelchair 1 and with the remote control 21, e.g. a smartphone.

The anchoring of the wheelchair 1 in the automobile 2 after being transferred inside before the start of the travel may be implemented, for example, by means of extendable mandrels or pins on the wheelchair 1 which, when the wheelchair 1 is positioned into place, fit into the floor or the part of the assembly located on the floor. Preferably, anchoring in multiple positions is possible so that the position of the wheelchair 1 relative to the steering wheel 20 can be adjusted according to the user's preference. The assembly may also include a seat belt buckle, for example if the buckle was located on the seat being replaced by the assembly in the original automobile 2, before the assembly was installed. The automobile 2 may be an automobile 2 without a B-pillar, as these automobiles 2 typically have more space in the open door 18, but generally any automobile 2 providing enough space for the transfer of a specific wheelchair 1 through the door 18 may be used. Thanks to the range of movements of the assembly, the support column 3 can also be placed, for example, behind the B-pillar or behind the back edge of the opening of the door 18. Thus, the support column 3 does not occupy part of the space of the door 18 and more space is available for movement of the wheelchair 1.

In some embodiments, the assembly further comprises an auxiliary frame. The auxiliary frame is attached to the wheelchair 1 and the attachment 5 is attached thereto. It may also comprise connectors for data or power connection of the wheelchair to the assembly, especially if suitable connectors are not directly on the wheelchair 1.

The method for transporting the wheelchair 1 for transporting persons with disability to the automobile 2, which is preferably implementable by the assembly of the invention, or by the automobile 2 with the assembly of the invention, comprises at least the following steps:
- Opening the door 18 of the automobile 2. The opening can be manual or mechanized as mentioned above. The door 18 may be rotational hinged or sliding. Preferably, the door 18 is secured against spontaneous movement after the opening.
- Approach of the wheelchair 1 to the open door 18, wherein the back side of the wheelchair 1 faces the automobile 2. The back side of the wheelchair 1 is the backward facing side in the standard direction of travel. The approach of the wheelchair 1 may be substantially perpendicular to the longitudinal axis 19 of the automobile 2, for example within a range of 90 ± 20°. In general, the direction of the approach of the wheelchair 1 may be substantially arbitrary, depending on the place of attachment of the arm 4 to the wheelchair 1, the structure of the wheelchair 1 and the automobile 2, etc.
- Extension of the arm 4 from the automobile 2 through the open door 18, wherein this arm 4 is attached slidably in the vertical and horizontal direction to the support column 3 and rotationally relative to the floor of the automobile 2. The support column 3 is fixed to the floor of the automobile 2 and the arm 4 is immovably connected to the wheelchair 1 by means of the attachment 5. The form of the arm 4, the support column 3, the movements thereof, the attachment 5, etc. may be in particular as described above for the assembly for transporting the wheelchair 1 for transporting persons with disability to and from the automobile 2. The connection of the wheelchair 1 to the attachment 5 may also include a data connection between the operating unit 16 of the wheelchair 1 and the control unit 12 of the assembly, in particular if the connection is wired. Alternatively, a wireless connection can be established, for example, as soon as the door 18 is opened or as the wheelchair 1 is brought closer to the automobile 2.
- The transfer of the wheelchair 1 by the arm 4 to the automobile 2, wherein the transfer comprises at least lifting the wheelchair 1, transferring the wheelchair 1 closer to the support column 3, and rotating the wheelchair 1. These movements are preferably controlled by the control unit 12 along a preset trajectory as mentioned above. Preferably, these movements at least partially overlap. A part of this step can also be sensing the position of the parts of the user's body and/or parts of the wheelchair 1 and stopping the movements in case the control unit 12 predicts an imminent collision.

One or more of the following steps may follow: transferring the wheelchair 1 in the automobile 2, e.g. to achieve a comfortable position relative to the steering wheel 20; placing the wheelchair 1 on the floor; anchoring the wheelchair 1 in the automobile 2; closing the door 18 (implemented preferably at least partly mechanically); connecting the power connector of the wheelchair 1 to the source in the automobile 2. The attachment of the wheelchair 1 may, in some embodiments, comprise a movement of the arm 4 to a precise position for attachment after the user has backed the wheelchair 1 alongside the automobile 2 within the reach of the assembly. The assembly may then further comprise a sensor of the position of the wheelchair 1, for example a camera, and the control unit 12 is adapted to move the arm 4 based on the data from the sensor of the position of the wheelchair 1 and to move the attachment 5 into the correct position on the wheelchair 1.

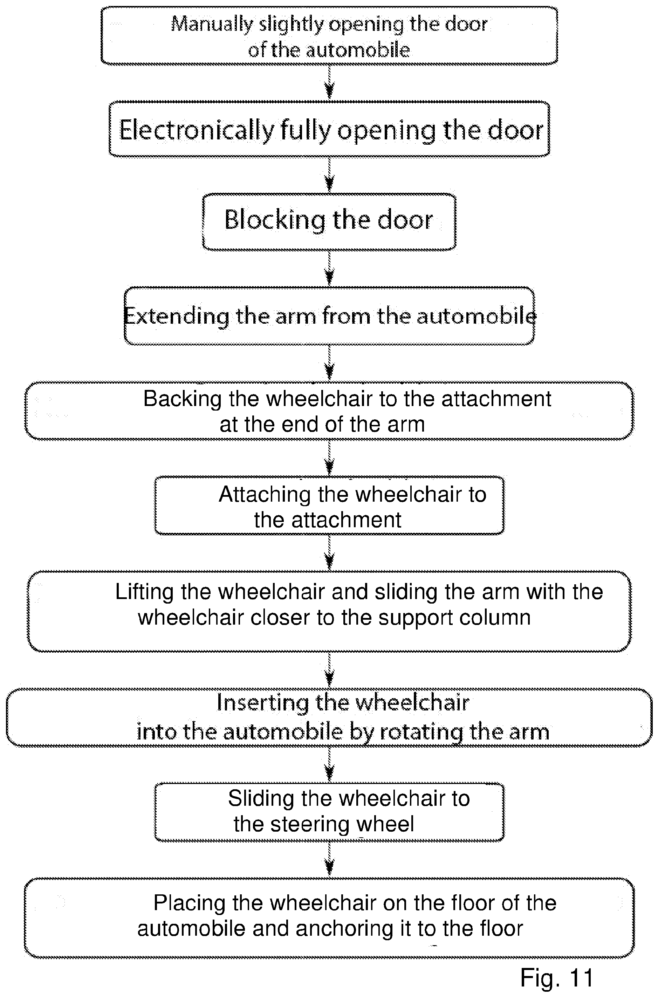

An exemplary embodiment of the method of the invention is schematically shown in fig. 11. In this method, the door 18 is slightly opened manually by the user and subsequently fully opened electronically by the user's instruction. The instruction can be given e.g. by the remote control 21 or voice assistant. Subsequently, the door 18 is blocked by the mechanism 17 for blocking the movement of the door 18, which may occur automatically or again based on the user's instruction. Subsequently the arm 4 is extended and rotated outward to a position suitable for attaching the wheelchair 1. This step is also preferably initiated by the user such that there is no risk of automatic extension of the arm 4 when the user is in the space in front of the door 18. The assembly can also include a sensor 14 for detecting the presence of persons or objects in the space of the door 18, wherein the control unit 12 prevents the arm 4 from extending if it is in danger of hitting a person/object.

Subsequently, the user of the wheelchair 1 backs the wheelchair 1 with the counterpart toward the attachment 5 on the arm 4 and the wheelchair 1 is attached to the arm 4. Securing with a lock can also be a part of the attachment. Subsequently, the user gives the instruction to initiate the transfer of the wheelchair 1 to the automobile 2. During the transfer, the wheelchair 1 is lifted from the ground, moved closer to the support column 3 and subsequently or concurrently the wheelchair 1 is rotated toward the automobile 2. After the wheelchair 1 is transferred into the automobile 2, the wheelchair 1 is adjusted to a position suitable for driving, in particular it is transferred closer to the steering wheel 20. Subsequently the wheelchair 1 is positioned on the floor, wherein it can land on it with its wheels and/or frame. For example, a part of the assembly on the floor may be adapted for landing and anchoring the frame of the wheelchair 1. Subsequently, the wheelchair 1 is anchored, the door 18 is closed, and the wheelchair 1 is ready for travel in the automobile 2.

The method for transporting the wheelchair 1 for transporting persons with disability from the automobile 2 may then comprise analogous steps in reverse order, in particular the door 18 may be opened, the wheelchair 1 transferred out of the automobile 2 by a combination of rotations and slides, the arm 4 disconnected from the wheelchair 1, etc.

The assembly of the invention preferably comprises a wheelchair 1 for transporting persons with disability, the structure of which facilitates the use of the assembly and facilitates the transfer of the wheelchair 1 to and from the automobile, anchoring the wheelchair to the floor, adjusting it into a position suitable for driving, communicative connection of the wheelchair 1 with the assembly or the automobile, etc.

An example of such a wheelchair 1, which is a preferable part of the assembly of the invention, is a wheelchair 1 for transporting persons with disability, which includes a chassis 22, a seat 25 with a frame 31 of the seat, a backrest 26 with a frame 30 of the backrest, a footrest 27, an armrest 44, a headrest 45, a travel drive 29, and a power source 28 for this drive. The chassis 22 comprises two axles, each fitted with a pair of wheels. The frame 31 of the seat supports the seating area, preferably provided with a padding, and is movably connected to the frame 30 of the backrest, which preferably also supports the padding. The connection between the seat 25 and the backrest 26, implemented by the movable connection between their frames, allows the inclination of the backrest 26 to be adjusted according to the user's needs and preferably also allows the wheelchair 1 to be folded by tilting the backrest 26 forward onto the seat 25. This movable connection may be rotational, or it may be a combination of a rotational and sliding movement, implemented by, for example, rotation of one frame on pins slidable on the other frame. In some embodiments, this connection may comprise a plurality of joints or rotational connections to ensure better tilting of the backrest onto the seat, e.g. to achieve a greater compactness of the wheelchair when it is fully folded.

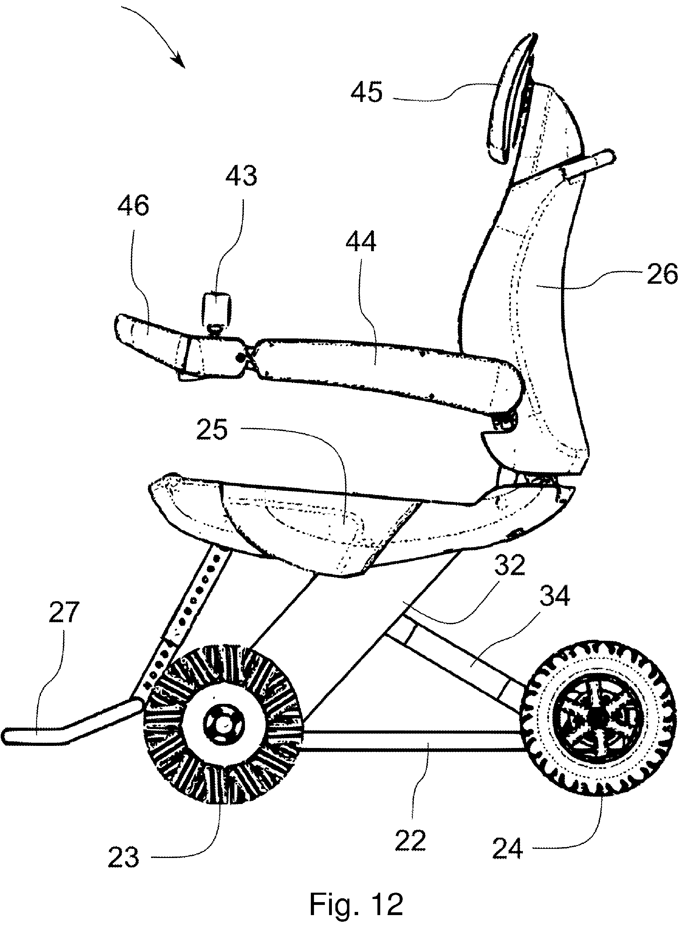

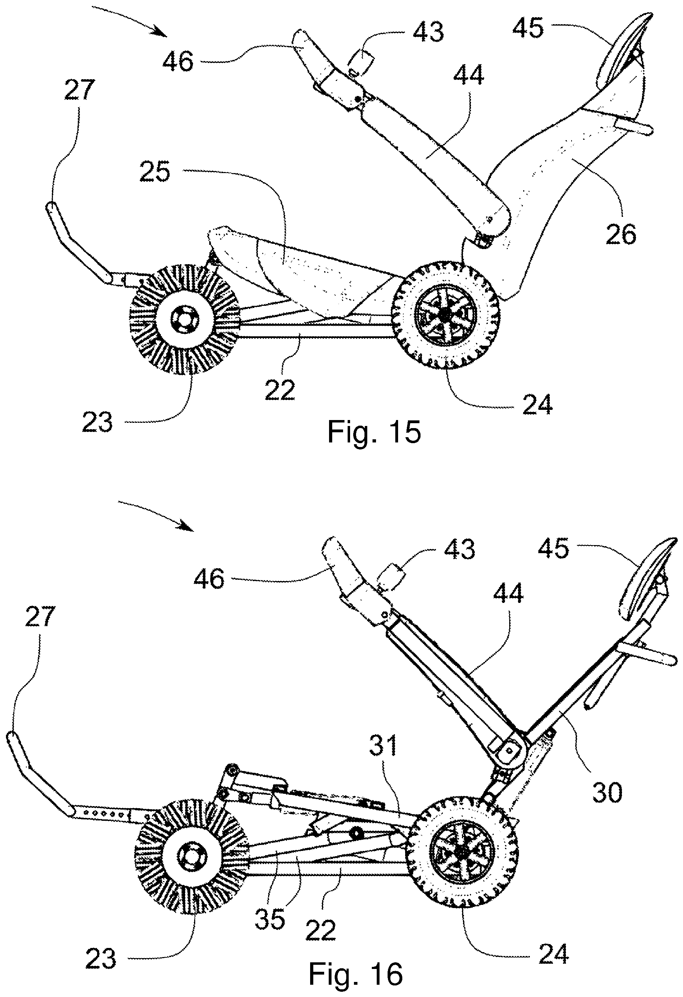

Furthermore, the frame 31 of the seat is movably connected to the footrest 27 to allow positioning of the footrest. The wheelchair 1 further comprises a positioning arm 32 that connects the frame 31 of the seat to the chassis 22. The positioning arm 32 is connected at its upper end to the frame 31 of the seat by at least one rotational connection and at its lower end, it is connected to the chassis 22 by at least one rotational connection at the front third, preferably quarter, of the length of the chassis 22. Preferably, the rotational connection 33 of the positioning arm 32 to the chassis 22 is located between the front wheels 23. The movement of the positioning arm 32, and thus the movement of the chassis 22 relative to the seating part of the wheelchair 1, is provided by a positioning linear drive 34, which is attached at the bottom end to the chassis 22, closer to the back wheels 24 than the positioning arm 32, and is attached at the upper end to the positioning arm 32. The linear drive is rotationally connected at both ends and when it is initiated, the two ends move away from each other or closer together. As a result, the inclination of the positioning arm 32 relative to the chassis 22 is adjusted, and thus the seat 25 and with it also the backrest 26, and other parts connected to it that form the seating part of the wheelchair 1, slide up or down. The wheelchair 1 can thus be adjusted between its positions, whereby in the standard position (see e.g. figs. 12 and 2) the seating part is in a position suitable for normal travel, where the backrest 26 is upright and the seat 25 is about half a meter above the ground, and in the lowered position the seat 25 is closer to the ground and the back wheels 24, the backrest 26 is tilted back and the footrest 27 can be slightly lifted (see e.g. figs. 15 and 16). Generally, in the standard position the distance of the seat 25, e.g. the lowest positioned point of the seating area, from the ground is at least 45 cm, preferably at least 50 cm, and in the lowered position of the wheelchair 1 this distance is a maximum of 30 cm, preferably a maximum of 25 cm. In the lowered position, the distance of the highest positioned point of the seat 25, at least with some slide of the seating area or backrest 26, from the ground may be a maximum of 50, more preferably a maximum of 45, and even more preferably a maximum of 40 centimeters from the ground.

When moving down, the seating part is moved backward at the same time. When viewed from the side, the frame 31 of the seat, the positioning arm 32, and the chassis 22 essentially define the shape of the letter "Z". The distance of the pair of the front wheels 23 from the pair of the back wheels 24 does not change during the positioning of the wheelchair 1, i.e. in particular the positioning mechanism is not implemented as a scissor mechanism. Preferably, the wheelchair 1 is also adjustable to other positions, in particular a plurality of positions between standard and (maximally) lowered, as well as to a raised position, which may be suitable for example for the user's work at kitchen counter and similar elevated work surfaces, and where the seat 25 may for example be more than 50 or more than 60 cm above the ground. Preferably, in all positions, the center of gravity of the wheelchair 1 is located between the front and back axles when viewed from above. The length of the wheelchair 1 is preferably for example a maximum of 150 cm. Its width is for example a maximum of 70 cm, more preferably a maximum of 60 cm. The speed of the wheelchair 1 can be limited to, for example, 15 km/h. The source 28 preferably provides a driving range of at least 20 km.

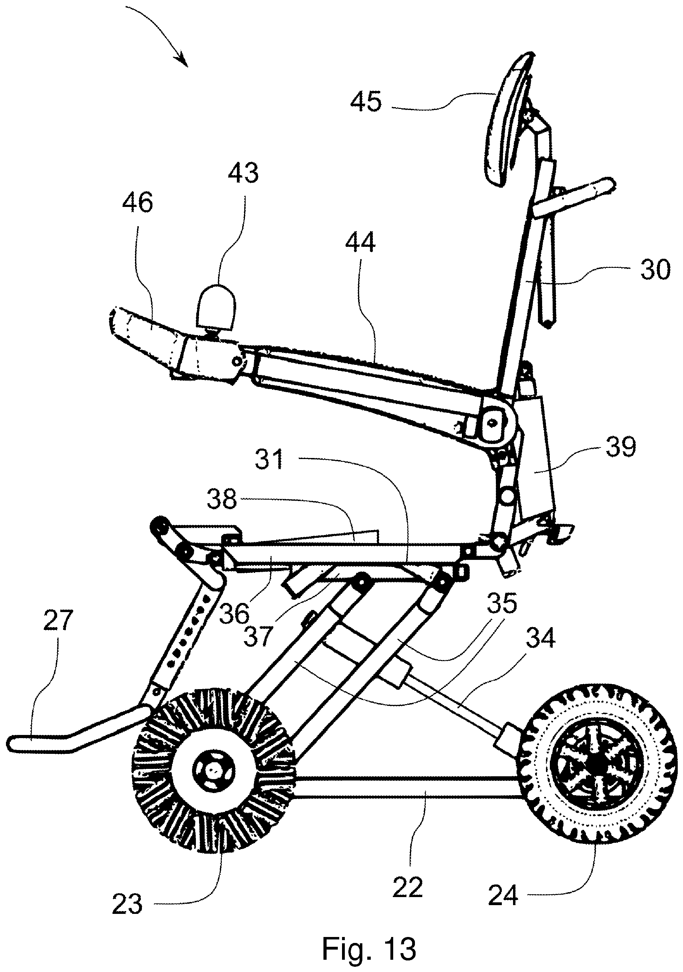

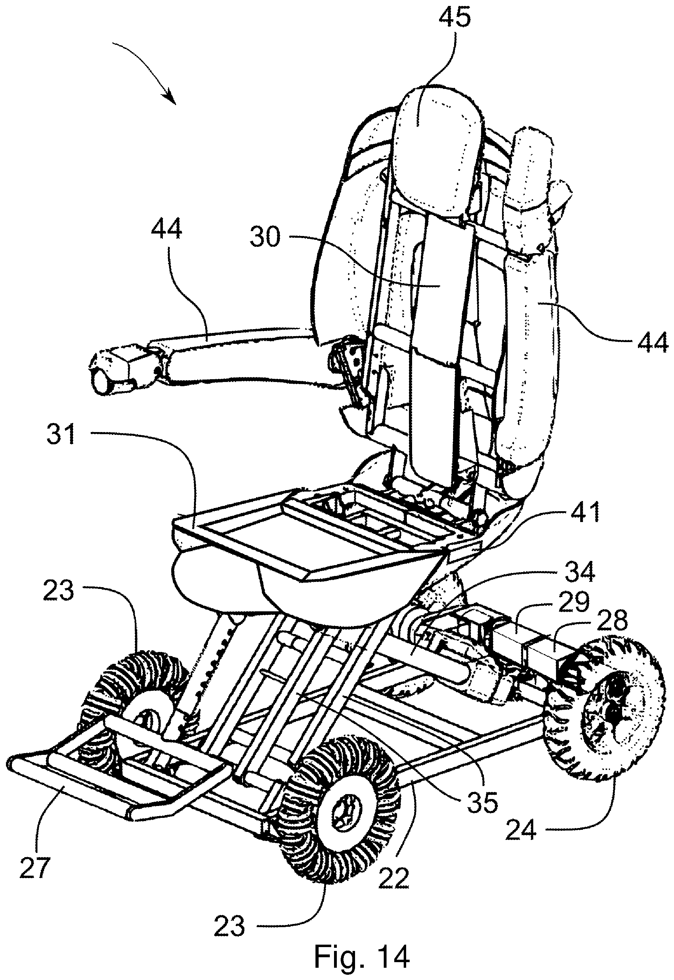

The chassis 22 may be made of, for example, metal profiles and/or composite materials, and preferably supports, in addition to the wheels, the positioning arm 32 and the positioning linear drive 34, the power source 28 and the travel drive 29 of the wheelchair 1, in particular the battery and the electric motor. Due to the weight of these components, it is more preferable to place them on the chassis 22 due to the stability of the wheelchair 1 and the energy requirement of positioning the seat 25. Furthermore, the chassis 22 may support, for example, brakes, a control unit 42, a gear train connecting the travel drive 29 to the driven wheels, preferably the back or all wheels, etc. Similarly, the frame 31 of the seat and frame 30 of the backrest, the positioning arm 32, and/or the rests may be made of metal profiles or plates as well. For example, duralumin or other aluminum alloy is preferably used to ensure a relatively low weight of the wheelchair 1. The arrangement of these components forming the framework of the wheelchair 1 can be seen, for example, in figs. 13 and 14. The wheelchair 1 preferably further comprises covers surrounding these support parts to ensure safety, protect these parts, and provide a user-friendly design. The positioning linear drive 34 may for example be implemented as a threaded rod connected to an electric motor, wherein this rod is attached to the positioning arm 32 through a housing with a complementary internal thread which is moved by rotating the rod. Alternatively, however, a pneumatic or hydraulic drive can be used. The positioning linear drive 34 is preferably attached to the chassis 22 in the back quarter of the chassis 22, preferably between the back wheels 24. Preferably, the chassis 22 defines a ground clearance of the wheelchair 1 of at least 50 mm, more preferably at least 60 mm.

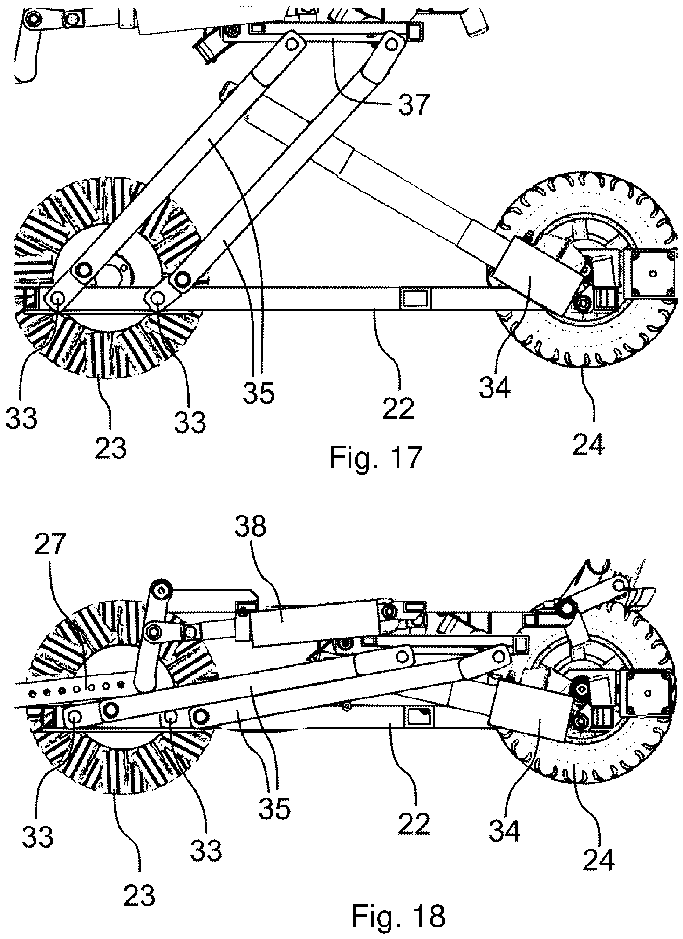

The positioning arm 32 preferably comprises a parallelogram comprising two profiles 35 of the arm parallel to each other and offset in the longitudinal direction of the wheelchair 1, i.e. in the standard direction of travel (see fig. 13). Each of these profiles 35 of the arm is rotationally connected to the chassis 22 or the frame 31 of the seat at both ends. On the chassis 22, both of these connections are preferably on the front quarter of the chassis 22, more preferably both are between the front wheels 23. The profiles 35 of the arm may be attached to the chassis 22 and/or frame 31 of the seat directly, or via additional connecting holders (see e.g. fig. 17), where the profile 35 of the arm is rotationally connected to the holder that is rotationally connected to the chassis 22 / frame such that a parallelogram is formed with certain clearances to allow smoother movement. The wheelchair 1 may comprise several such parallelograms, for example two on the sides of the wheelchair 1, but it may also comprise only one. The profiles 35 of the arm can then be doubled to ensure higher firmness (see e.g. fig. 14).

The footrest 27 is, in the shown embodiment, formed of metal profiles arranged in an approximate rectangle (see fig. 14) and is attached to the frame 31 of the seat by a telescopic rod that is rotational relative to the frame 31 of the seat - the adjustability of the footrest is apparent, for example, when comparing figures 2, 4, and 5 and a detail of its movable attachment is in figs. 18, 12, and 13. In an exemplary embodiment, the movement of the footrest 27 is preferably provided by a drive 38 for rotating and/or extending the footrest 27, such as a linear drive (e.g. a gear rack, threaded rod, hydraulic or pneumatic piston, etc.). In the shown embodiment, the telescopic rod of the footrest is provided with a rod at the end to which it is firmly connected at an obtuse angle, wherein this rod is rotationally connected to the frame 31 of the seat and the drive 38 engages it for rotating and/or extending the footrest 27. This drive is built into the seat 25 and powered by the source 28. For example, the length of the telescopic rod can be adjusted manually. In some embodiments, the rotation of the footrest can also be manually adjusted. The adjustment of the footrest 27 may be independent of the adjustment of other parts of the wheelchair 1, or it may be coupled with the adjustment of some other part, e.g. the footrest 27 may be lifted when the backrest is tilted or transferred to a lowered position.

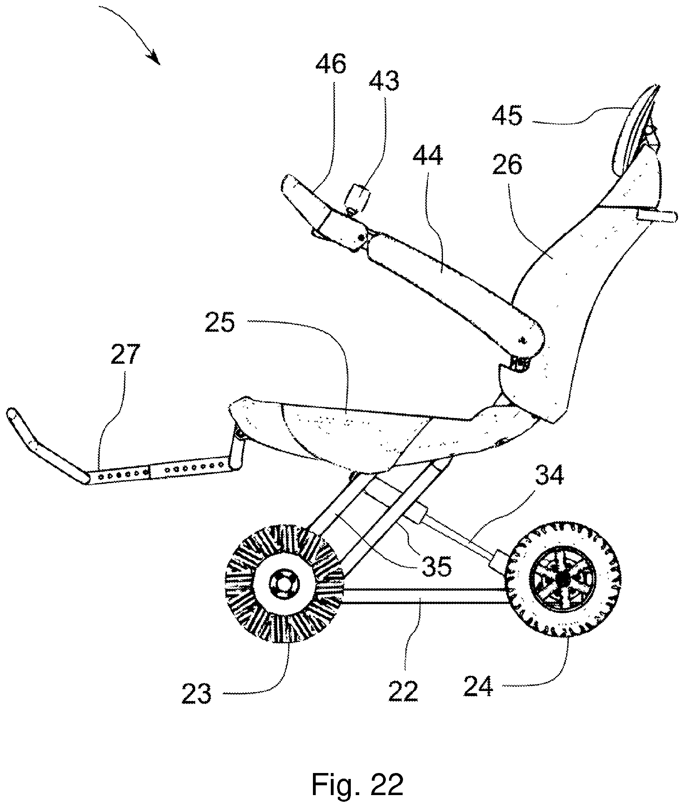

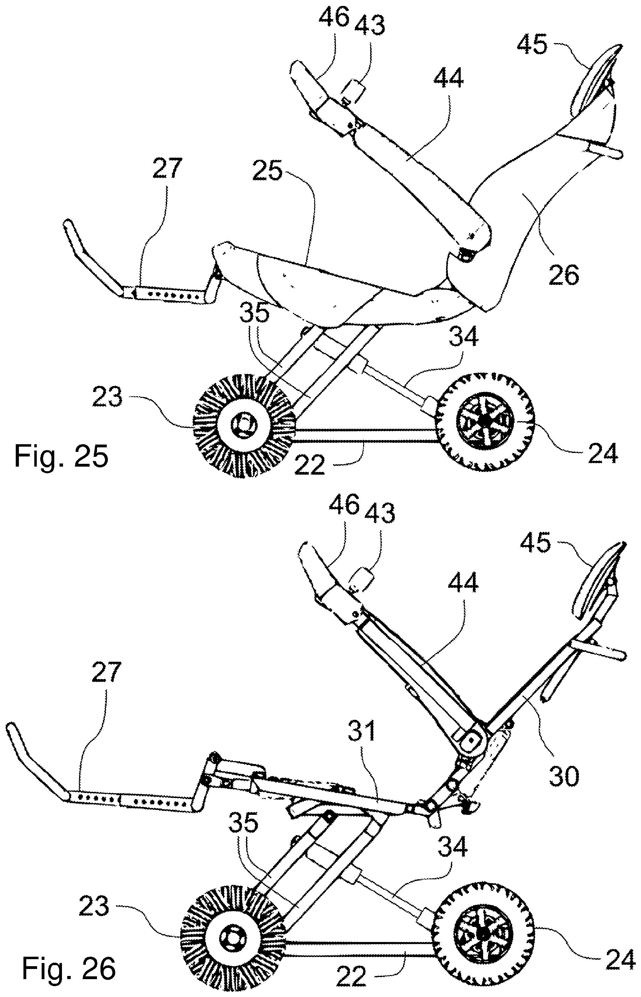

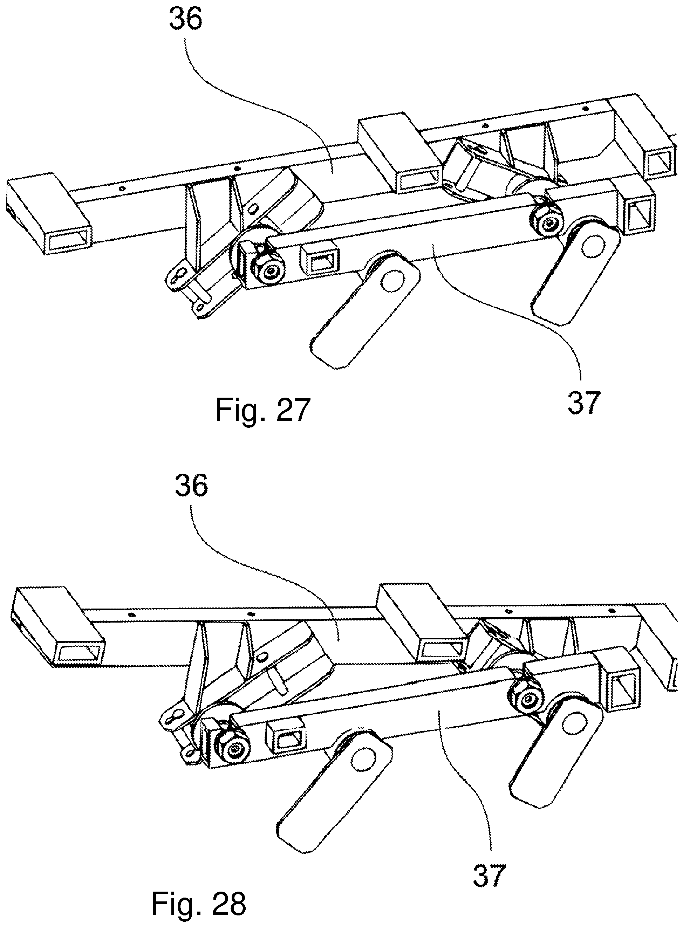

The frame 31 of the seat defines the approximately rectangular plan of the seat 25, provides the connection between the seating part of the wheelchair 1 (in particular the seat 25, the backrest 26, the footrest 27, the armrest 44, the headrest 45) and the drive part of the wheelchair 1 (in particular the positioning arm 32, the chassis 22, the drive, and the source 28) and serves to attach the footrest 27 and the backrest 26. A drive for adjusting the footrest 27, as described above, and, for example, a drive 39 for rotating the backrest 26 or tilting the seating area, as will be described later, can be built into it. Preferably the frame 31 of the seat comprises a top piece 36 and a bottom piece 37, which can be tilted relative to each other. The top piece 36 supports the seating area and the bottom piece 37 is connected to the positioning arm 32. Thus, the tilting of the top piece 36 ensures the tilting of the seating area (see figs. 25, 26 compared to e.g. fig. 22). The footrest 27 and backrest 26 may be connected to any of these pieces, and in the shown embodiment, they are attached to the top piece 36 such that they are moved when adjusting the inclination of the seating area. The connection of the top piece 36 to the bottom piece 37 may be rotational or rotationally slidable. For example, they may be connected by a pin. In the shown embodiment, they are connected by the mechanism shown in figs. 27 and 28, where the top piece 36 comprises two mutually non-parallel rails on each lateral side, in which sliding elements - pins, which are rotationally connected to the bottom piece 37 - are slidably attached. The movement of the sliding elements in the rails, due to the inclination of the rails, modifies the inclination of the top piece 36 - adjusts its rotation around a horizontal axis perpendicular to the longitudinal direction of the wheelchair 1. The range of this rotation may be, for example, 0±10°, where zero rotation corresponds to the parallel position of the top piece 36 with the bottom piece 37, i.e., substantially horizontal position of the top piece 36. Preferably, the tilting of the seating area of the seat 25 is provided by a drive 40 for tilting the top piece 36, e.g. an electronic drive. It may be a rotatory drive or a linear drive, engaging at one end the top piece 36 and at the other end the bottom piece 37. This drive is preferably built into the frame 31 of the seat. The power supply is preferably provided from the source 28 on the chassis 22, for example a power cable for all electronic components on the seating part of the wheelchair 1 may be routed on the positioning arm 32. This tilting may be independent of the adjustment of the other parts or may be coupled with the adjustment of the other parts.

The frame 30 of the backrest may be rotationally connected to the frame 31 of the seat, for example by a hinge, or by a combination of rotational and sliding movement. It may comprise metal profiles defining the shape of the backrest 26, elements for supporting the lumbar spine, etc. Rotation of the frame 30 of the backrest relative to the frame 31 of the seat is preferably provided by the drive 39 for rotating the backrest 26 (see fig. 13), for example by a linear drive engaging at one end the seat 25 and at the other end the backrest 26. In the shown embodiment, this drive is built into the backrest 26, but it may also be in the seat 25. The range of rotation of the backrest 26 preferably allows a tilting backward of at least 30° relative to the vertical position (see figs. 15 and 16) and allows a tilting forward preferably until the backrest 26 sits on the seat 25 such that the wheelchair 1 can be folded as much as possible. A handle may be attached to the frame 30 of the backrest, allowing possible manual handling of the wheelchair 1, and the headrest 45 and the armrest 44 on both lateral sides are attached thereto. The backrest 26 may be provided with light elements, e.g. to provide visibility from behind or to indicate a change in the direction of travel or to illuminate the space next to the wheelchair 1. For example, brake lights or parking lights may also be included. Further, a central switch of the wheelchair 1 may be located on the backrest 26 or the chassis 22 for disconnecting all electronic components of the wheelchair 1 from the source 28.

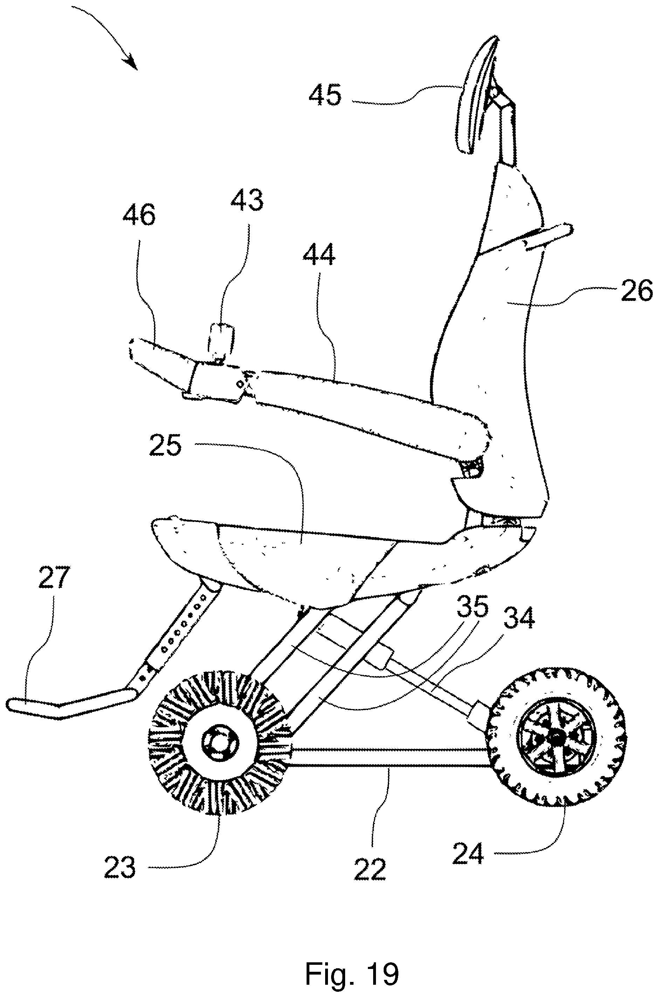

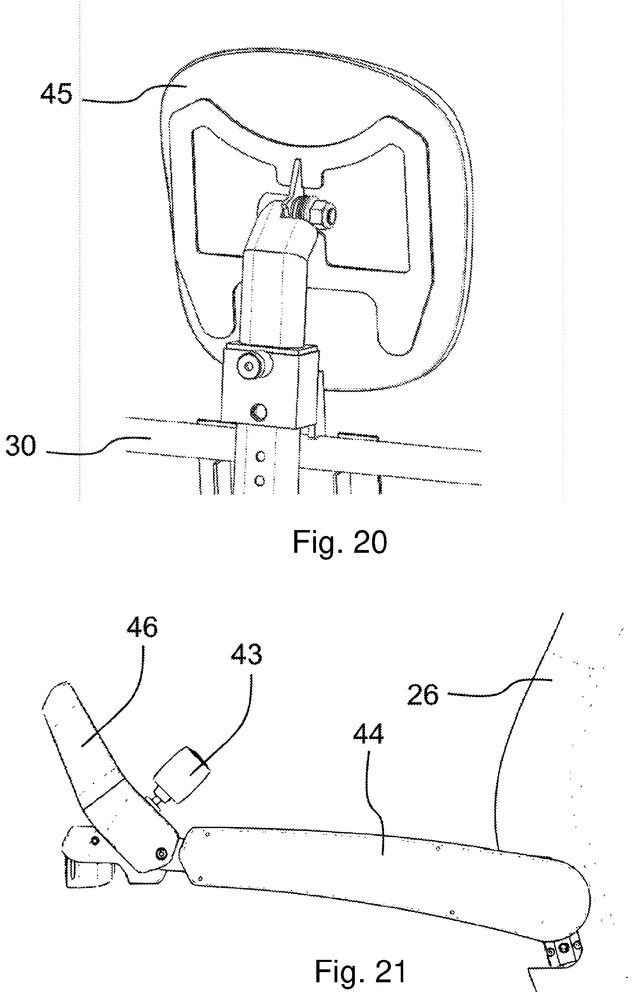

The headrest 45 can be preferably extended from the backrest 26 in the vertical direction and simultaneously it is preferably rotational around the horizontal axis perpendicular to the longitudinal direction of the wheelchair 1. The adjustment of the headrest 45 by these movements may be provided by a drive or drives, in the shown embodiment it is carried out manually, wherein it is ensured by elastic or frictional elements that the headrest remains in the position to which it is manually adjusted. The extended state of the headrest 45 is shown in fig. 19, a detailed view of the slidable and rotational attachment of the headrest is in fig. 20. As can be seen in fig. 20, the headrest 45 may comprise a frame in the form of a metal plate to which a functional part of the headrest, such as a cover with padding, is attached.

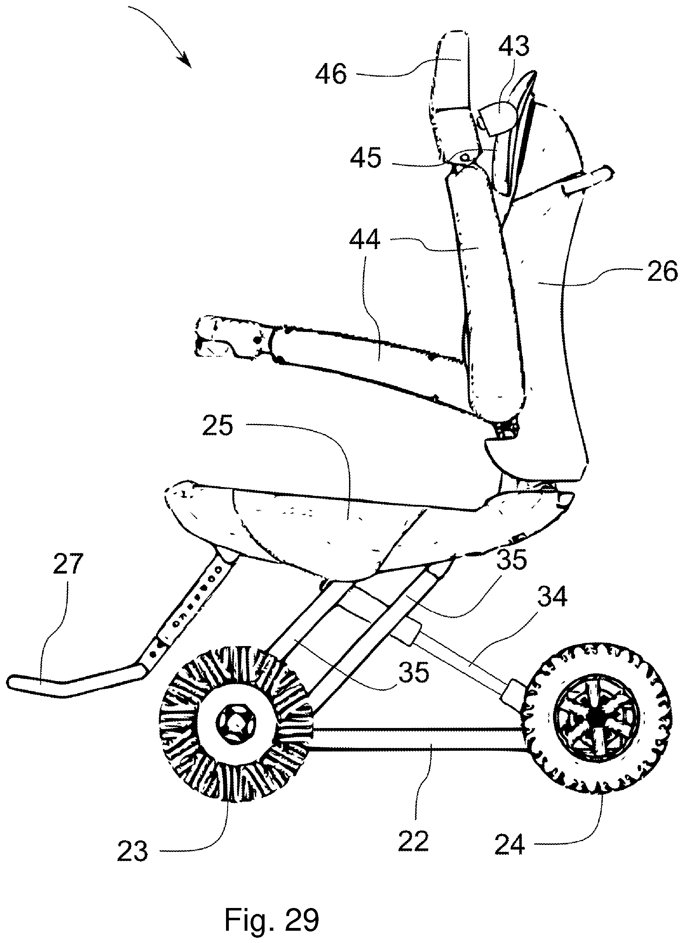

The armrests 44 are preferably both rotational by at least 90° such that they can be rotated up to facilitate getting off the wheelchair 1 and getting on it (see fig. 29, where the left rest is raised, and the right one is left in the standard horizontal position). They can be rotationally connected to the frame 30 of the backrest. In the exemplary embodiment, these armrests are rotationally attached on a joint or pin that is slidable in a rail or other slidable guide on the frame 30 of the backrest, wherein said rail is preferably substantially vertical in the standard position of the wheelchair 1. This allows the armrests 44 to be height-positioned by sliding them in these rails. The armrest 44 may comprise a metal frame provided with a cover and/or padding. In the shown embodiment, one of the armrests 44 further comprises operating elements 43, e.g. a joystick for operating the travel drive 29 and rotating the wheelchair 1, a lever for braking, buttons or sliders for adjusting the individual parts of the wheelchair 1, voice control, etc. The rotation may be provided in particular by rotating one wheel of the driven wheels pair faster than the other or by rotating these wheels in opposite directions. For example, the driven wheels are in the back and the front wheels 23 are omnidirectional to ensure smooth rotation in a relatively small space. Preferably, for example, the width of rotation at a point is a maximum of 200 cm, more preferably a maximum of 180 cm. The radius of rotation (during the travel) is preferably a maximum of 300 cm, more preferably a maximum of 280 cm.

The armrest 44 may further comprise a display device 46, i.e. in particular a display, for displaying information about the wheelchair 1 or the user, such as state of charge, maximum driving range, etc. It is also possible, for example, to display data from parking sensors or camera to ensure safe backing up of the wheelchair 1, to check the attachment of the arm to the wheelchair 1 (or the auxiliary frame, as will be discussed below), etc. The display device 46 may also serve as an operating element 43, in particular, a touch screen may be used or it may be possible to attach a smartphone or other user device 47 on the armrest 44 and communicatively connect it to the control unit 42 of the wheelchair 1 for displaying information on the phone and/or operating the wheelchair 1 by the phone. The armrests 44 may also be provided with, for example, a light for illuminating the ground in front of the wheelchair 1, an acoustic signalization, etc. A detailed view of one of the armrests 44, provided with the display device 46 and the operating element 43, is shown in fig. 1.

The wheelchair 1 preferably further comprises a control unit 42, data connected to the drives, light sources, acoustic signalization source, power source 28, brakes, operating elements 43, display device 46, etc. However, more preferably, the operation of the brakes may be alternatively or additionally provided mechanically so that it is not dependent on the electronics of the wheelchair 1 or the state of charge of the source 28 in any way. For example, there may be an operating brake on the wheelchair 1, which can be controlled electronically, and a parking brake, which is independent of the electronics. The control unit 42 can therefore implement the adjustment of the individual parts of the wheelchair 1 with respect to the user's instructions received by the operating elements 43, provide movement of the wheelchair 1 by controlling the travel drive 29 or braking the wheels, perform diagnostics of the wheelchair 1 or diagnostics of the user if the wheelchair 1 is provided with diagnostic sensors, etc. For example, in some embodiments, the control unit 42 may be adapted to couple the movement of some of the drives, for example to tilt the backrest 26 and lift the footrest 27 when the wheelchair 1 is being transferred to the lowered position. These movements can be coupled also with the tilting of the seating area in some embodiments. An advantage of coupling these movements by the control unit 42 instead of, for example, mechanical coupling by rods is the possibility of deactivating the coupling with respect to the user's requirements.

The wheelchair 1 may further comprise an auxiliary frame, which is adapted to implement the connection (mechanically and/or electronically) of the wheelchair 1 with the arm of the assembly for transporting the wheelchair. For example, the auxiliary frame can be a U-shaped profile that surrounds a part of the frame of the given wheelchair 1 from the back, it can be a plate attached to the backrest 26 of the wheelchair 1 from the back or to the seat 25 from below, etc.

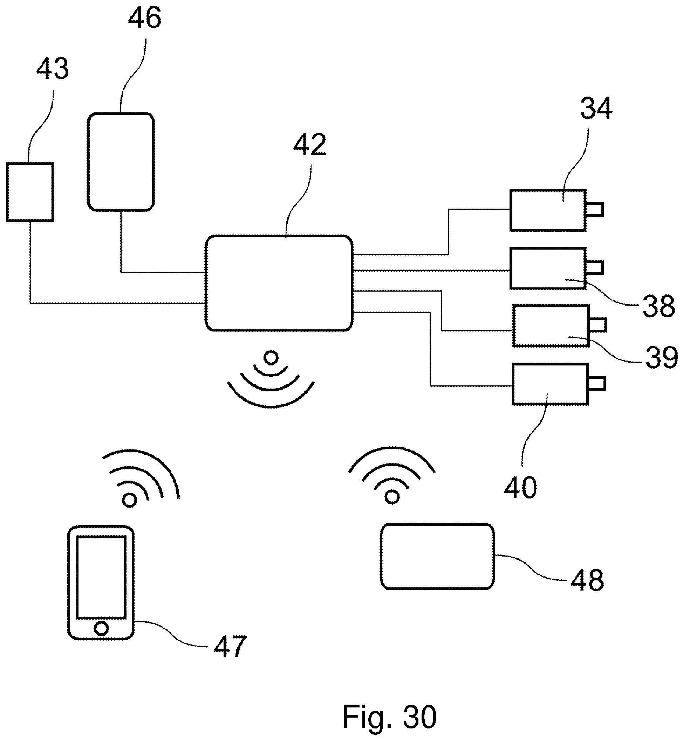

Fig. 30 schematically shows data connection between the control unit 42 and the drives, the operating elements 43, and the display device 46, and also shows the wireless communicative connection with the user device 47 and the control unit 48 of the assembly for transferring the wheelchair 1 to and from the automobile. To enable transfer by such an assembly, the wheelchair 1 may further comprise an attachment 10 for attaching an arm of the assembly, for example an opening into which a complementary end of the arm fits for attaching the wheelchair 1 and allowing it to be lifted and transferred between the exterior and interior of the automobile. The control unit 42 of the wheelchair 1 may then also enable a communicative connection with the control unit of the automobile, and preferably the wheelchair 1 is provided with a connector for recharging the source 28 from the automobile during the travel, for data connection with the automobile, etc.

The control unit 42 may also be adapted to remotely control the wheelchair 1, without a seated user, for example via the user device 47 wirelessly communicatively connected to the control unit 42.

In alternative embodiments, some of the movements of some of the parts of the wheelchair 1, which are provided by drives in the shown embodiment, may be realized manually and vice versa. For example, the armrests 44 or the headrest 45 can also be adjusted by the drive. Alternatively, for example, only the movement of the seat 25 relative to the chassis 22 may be operated electronically and the other movements are implemented manually. However, preferably at least the movement of the seat 25 relative to the chassis 22 and the movement of the footrest 27 and the backrest 26 are automated (electronically, pneumatically, etc.). In some embodiments, the control unit 42 may be adapted to operate at least the drive 38 for rotating and/or extending the footrest 27 and the drive 39 for rotating the backrest 26, preferably also the drive 40 for tilting the top piece, according to a predefined schedule, in particular in cycles, to provide automatic exercise or stretching of the user or to periodically change the position of their seating to prevent pressure sores.

In some embodiments, the front wheels 23 can also be the driven wheels. Embodiments are also possible in which the wheelchair 1 comprises more than four wheels, for example it may comprise an axle with auxiliary wheels in front of the front wheels 23 or behind the back wheels 24. The front wheels 23 or the back wheels 24 - in particular the non-driven pair of wheels, can also be individually rotational around the vertical axis in some embodiments.

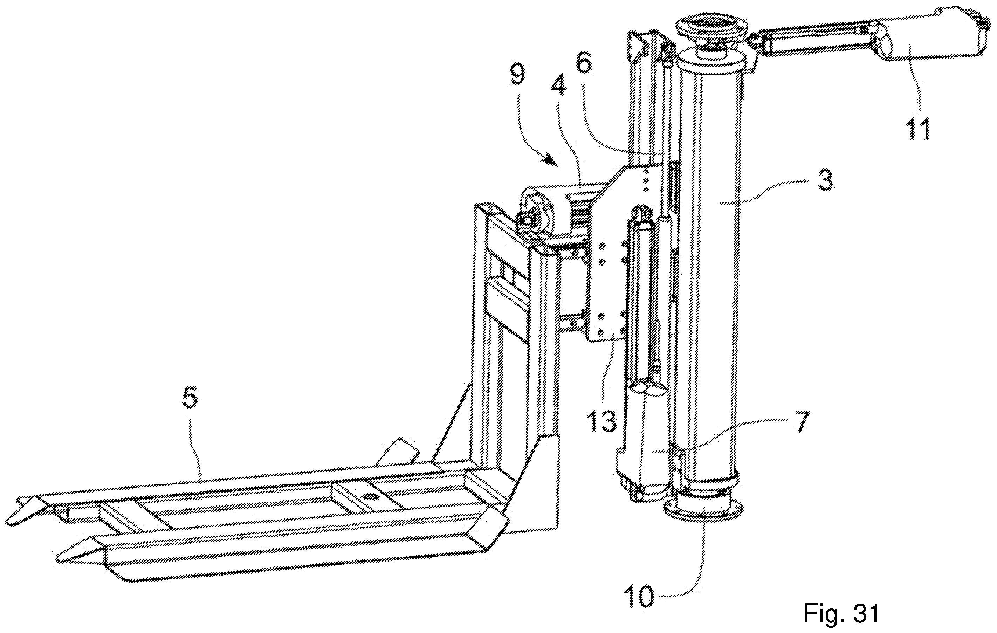

Figs. 31 to 36 show an alternative embodiment of the assembly for transporting the wheelchair 1 to and from the automobile 2. In this embodiment, the attachment 5 is implemented as a platform for the wheelchair 1 to be driven onto. Thus, a corresponding method for transporting the wheelchair 1 could comprise the step of driving the wheelchair 1 to the automobile 2 only after the step of extending the arm 4 and positioning the platform on the ground. In the shown embodiment, this assembly with the platform contains the wheelchair 1 implemented as described above. In general, however, it can also be implemented separately or with any other suitable wheelchair 1. An advantage of this embodiment may be the easier attachment of the wheelchair 1 to the arm 4 - driving onto the platform requires less precision while backing up than, for example, driving with an opening in the frame onto the sliding attachment 5. Simultaneously, the firmness of the attachment 5 and the stability of the wheelchair 1 during the transfer can be increased. Even in this embodiment, it may be preferable if the wheelchair 1 can be partially inserted into the space next to the support column 3, i.e., if the projection of the column is offset from the projection of the wheelchair 1 when viewed in the direction of the movement defined by the sliding drive 9, or if the sliding guide element 8 is placed next to the support column 3 when viewed in the axis of the column.

Conversely, an embodiment with the sliding attachment 5 or a similar attachment not comprising elements for driving the wheels of the wheelchair 1 onto the attachment 5 can transfer the wheelchair 1 even into an automobile 2 with a smaller door opening or a smaller space in front of the steering wheel 20 due to a greater horizontal slide range and a shorter arm 4 with the attachment 5, compared to the embodiment with a platform.

In this embodiment, the basic features of the assembly are analogous to the embodiment described above - the assembly comprises the support column 3 attachable to the floor, and in this embodiment also to the ceiling of the automobile 2. The arm 4 is attached to the column by the connecting element 13 slidably in the horizontal direction within a range of at least 10 cm, where the slide is provided by the corresponding linear guide and the sliding drive 9. For example, this drive can be adapted for a slide at a speed of 10-20 mm/s. Further, the connecting element 13 is vertically slidable on the column using the lifting guide element 6 and the lifting drive 7. The lifting can be in the range of, for example, 20-50 mm, e.g. 30-40 mm. The speed of the lifting can be for example 30-40 mm/s. The column with the arm 4 can rotate relative to the automobile 2, e.g. in a range of at least 90°. In this embodiment, the rotating drive 11 is placed near the ceiling and can be adapted, for example, for a speed of rotation of 5-15° per second. The linear drives here are, for example, telescopic, but threaded rods can also be used.

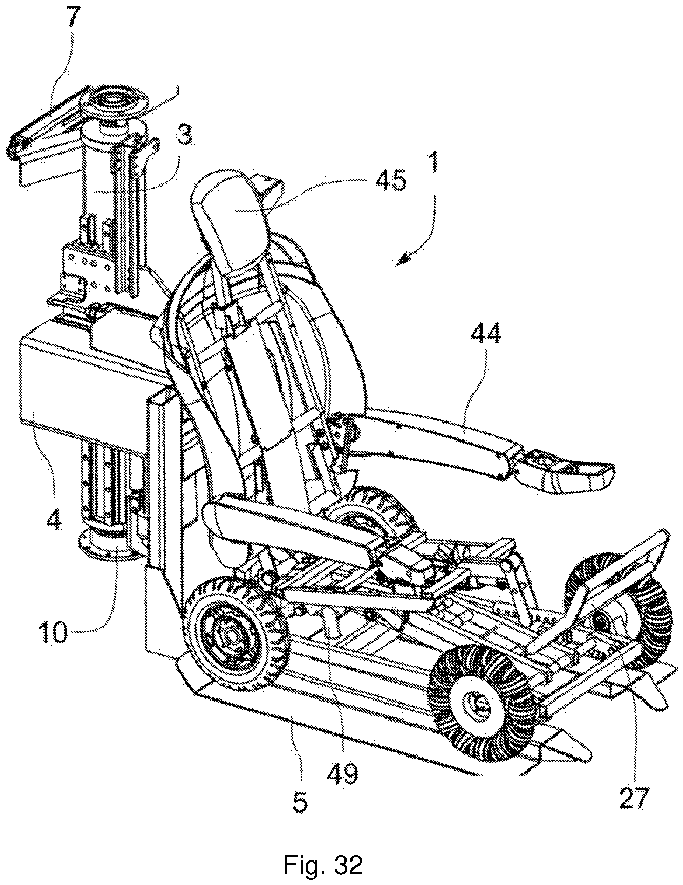

The platform may comprise a pair of profiles for the wheels of the wheelchair 1, wherein these profiles are firmly connected to each other and are firmly connected to the end of the arm 4 by the vertical part of the platform. As can be seen e.g. in fig. 32, these profiles for the wheels can be arranged such that the wheelchair 1 can be conveniently driven onto them after the platform has been positioned on the ground, e.g. by bending the front ends of the profiles such that they are flush with the ground. The back end of these profiles is raised to define a stop for the wheelchair 1. In the shown embodiment, the wheelchair 1 is anchored against unwanted movement off the platform by the securing pin 49 connecting the chassis 22 of the wheelchair 1 to the platform. Alternatively or additionally, for example, a similar pin or other lock may be placed on the vertical part of the platform for anchoring the backrest 26 or on the back of the seat 25. The wheelchair 1 may then also comprise the auxiliary frame which enables this anchoring even on the wheelchair 1 which was not adapted for this purpose in the manufacture. The platform may comprise, for example, sensors of the correct placement of the wheelchair 1 on the platform, sensors of parts of the user's body as described above, etc.

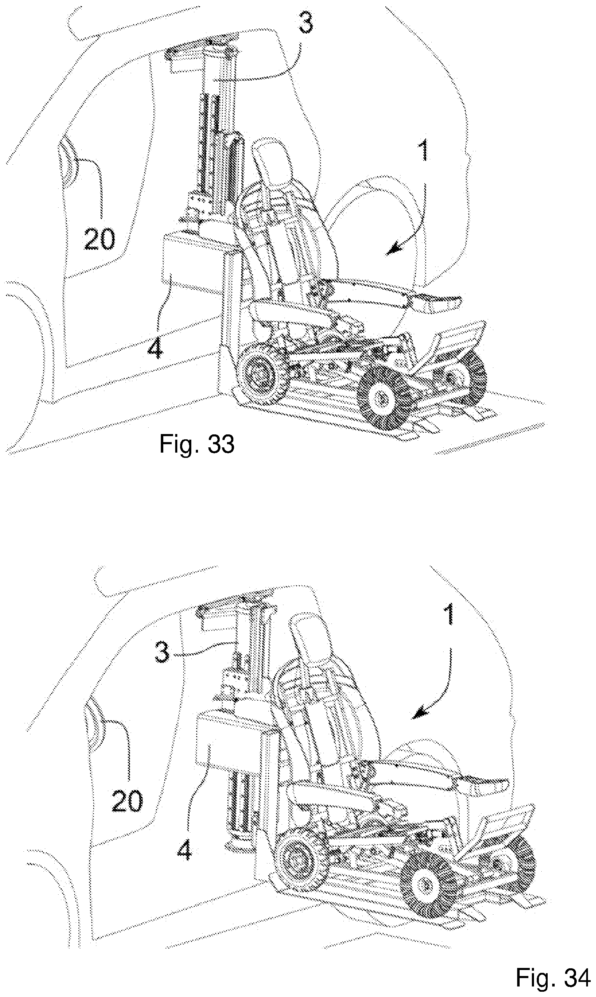

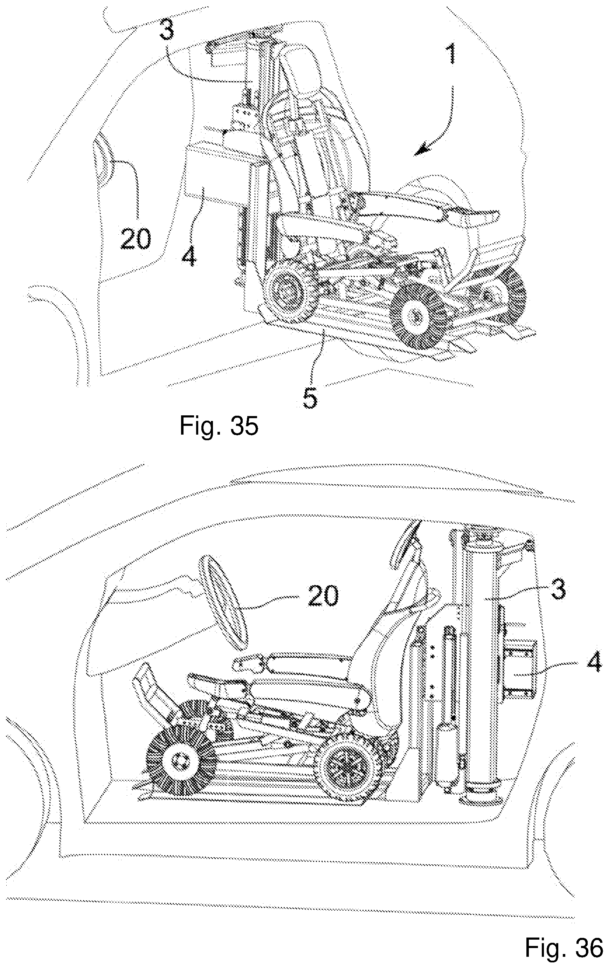

The movement of the wheelchair 1 during the transfer is then analogous to the movement from the embodiment mentioned above and can be seen by comparing figs. 33 to 36. After the wheelchair 1 has been driven onto the platform and the wheelchair 1 has been preferably anchored to the platform (fig. 33), the lifting of the platform is initiated (fig. 34) and subsequently the platform slides closer to the support column 3 (fig. 35) and is rotated, whereby the wheelchair 1 is transferred inside the automobile 2 (fig. 36). Subsequently, the platform can preferably be placed on the floor of the automobile 2, wherein it can also be transferred further away from the column closer to the steering wheel 20. After the wheelchair 1 is driven onto the platform, the wheelchair 1 may be lowered, i.e. the seat 25 may be brought closer to the wheels and the platform, before initiating the transfer or while the platform is being lifted, if the wheelchair 1 is adapted for such positioning. This reduces the risk of injury to the head during the transfer, or it allows the assembly to be used in automobiles 2 with lower doors. This positioning of the wheelchair 1 may be automatic, particularly in embodiments where the wheelchair 1 is communicatively connected to the assembly or the automobile 2. It can also be manual, i.e. user-executed, wherein the wheelchair 1 can then, for example, alert the user after driving onto the platform that the wheelchair 1 needs to be adjusted to a lowered position to be able to initiate the transfer. The assembly may comprise sensors that detect e.g. the height of the position of the headrest 45 and the transfer can then be initiated only when it is verified that the headrest does not extend beyond a certain height. Even for the embodiment with a platform, it is preferable when the assembly is placed in an automobile 2 with a sliding door. Regardless of the type of the door, the assembly preferably comprises a mechanism for blocking the movement of the door, unless this mechanism is already built into the automobile 2. Similarly, in an exemplary embodiment with a platform, it may be preferable if the control unit 12, 48 of the assembly can be used to operate a plurality of drives simultaneously, for example, to rotate the arm 4 while sliding it with the sliding drive 9. However, due to the size of the platform and the size of horizontal slide, it may be necessary to lift the platform fully above the level of the sill of the vehicle before initiating the transfer in a vertical plane. Further, in the embodiment with the platform, it is still preferably ensured that the assembly is located entirely on one side of the longitudinal axis 19 of the automobile 2.

**List of Reference Signs**

| | | | |
|---|---|---|---|
| 1 - | Wheelchair | 31 - | Frame of the seat |
| 2 - | Automobile | 32 - | Positioning arm |
| 3 - | Support column | 33 - | Rotational connection of the positioning arm to the chassis |
| 4 - | Arm | | |
| 5 - | Attachment | 34 - | Positioning linear drive |
| 6 - | Lifting guide element | 35 - | Profile of the arm |
| 7 - | Lifting drive | 36 - | Top piece |
| 8 - | Sliding guide element | 37 - | Bottom piece |
| 9 - | Sliding drive | 38 - | Drive for rotating and/or extending the footrest |
| 10 - | Rotating guide element | | |
| 11 - | Rotating drive | 39 - | Drive for rotating the backrest |
| 12 - | Control unit | 40 - | Drive for tilting the top piece |
| 13 - | Connecting element | 41 - | Attachment for attaching the arm of the assembly |
| 14 - | Sensor | 42 - | Control unit |
| 15 - | Communication unit | 43 - | Operating elements |
| 16 - | Operating unit of the wheelchair | 44 - | Armrest |
| 17 - | Mechanism for blocking the movement of the door | 45 - | Headrest |
| 18 - | Door | 46 - | Display device |
| 19 - | Longitudinal axis | 47 - | User device |
| 20 - | Steering wheel | 48 - | Control unit of the assembly |
| 21 - | Remote control | 49 - | Securing pin |
| 22 - | Chassis | | |
| 23 - | Front wheel | | |
| 24 - | Back wheel | | |
| 25 - | Seat | | |
| 26 - | Backrest | | |
| 27 - | Footrest | | |
| 28 - | Source | | |
| 29 - | Travel drive | | |
| 30 - | Frame of the backrest | | |

## Claims

1. An assembly for transporting a wheelchair (1) for transporting persons with disability to and from an automobile (2) comprising a support column (3) for attachment to a floor of the automobile (2), an arm (4) connected to the support column (3), and a drive mechanism for moving the arm (4), wherein the arm (4) is provided with an attachment (5) for attaching the wheelchair (1) to the arm (4), **characterized in that** the drive mechanism comprises a lifting guide element (6) with a lifting drive (7) for vertically sliding the arm (4) relative to the support column (3), a sliding guide element (8) with a sliding drive (9) for horizontally sliding the arm (4) relative to the support column (3) for changing radius of rotation of the wheelchair (1) around an axis of the support column (3) when the wheelchair (1) is attached to the attachment (5), and a rotating guide element (10) with a rotating drive (11) for rotating the arm (4) around the axis of the support column (3), wherein when viewed in direction of axis of the support column (3), axis of the sliding guide element (8) is offset from the support column (3), wherein the drive mechanism further comprises a control unit (12) adapted for operating the lifting drive (7), the sliding drive (9), and the rotating drive (11), wherein the drive mechanism comprises a connecting element (13) that is slidably attached on the support column (3) and moves together with the arm (4) when the arm (4) slides vertically, wherein the arm (4) is slidably attached to the connecting element (13) and the sliding drive (9) is adapted for sliding the arm (4) horizontally relative to the connecting element (13).

2. The assembly for transporting a wheelchair (1) for transporting persons with disability according to claim 1, **characterized in that** both the lifting guide element (6) and the sliding guide element (8) pass through the connecting element (13).

3. The assembly for transporting a wheelchair (1) for transporting persons with disability according to any one of the preceding claims, **characterized in that** when viewed in the axis of the support column (3), the sliding guide element (8) passes next to the support column (3).

4. The assembly for transporting a wheelchair (1) for transporting persons with disability according to any one of the preceding claims, **characterized in that** the control unit (12) is adapted to control at least two drives from the lifting drive (7), the sliding drive (9), and the rotating drive (11) simultaneously.

5. The assembly for transporting a wheelchair (1) for transporting persons with disability according to any one of the preceding claims, **characterized in that** for transporting the wheelchair (1) into the automobile (2) after attaching the wheelchair (1) to the attachment (5), the control unit (12) is adapted to control the drives for lifting the wheelchair (1), bringing the wheelchair (1) closer to the support column (3), rotating the wheelchair (1) around the column, moving the wheelchair (1) away from the support column (3), and placing the wheelchair (1) on the floor of the automobile (2).

6. The assembly for transporting a wheelchair (1) for transporting persons with disability according to any of the preceding claims, **characterized in that** it further comprises a mechanism (17) for blocking the movement of the door (18) of the automobile (2).

7. The assembly for transporting a wheelchair (1) for transporting persons with disability according to any of the preceding claims, **characterized in that** it further comprises an auxiliary frame for attachment to the wheelchair (1), wherein the attachment (5) is adapted for being attached to the auxiliary frame.

8. An assembly for transporting persons with disability comprising the assembly for transporting a wheelchair (1) according to any one of the preceding claims, **characterized in that** it further comprises a wheelchair (1) for transporting persons with disability comprising a chassis (22) provided with a pair of front wheels (23) and a pair of back wheels (24), a seat (25), a backrest (26), a footrest (27), a power source (28), and a travel drive (29), wherein the backrest (26) comprises a frame (30) of the backrest and the seat (25) comprises a frame (31) of the seat movably connected to the frame (30) of the backrest, to the footrest (27), and to the chassis (22), wherein the pair of the front wheels (23) is held at a constant distance from the pair of the back wheels (24) by the chassis (22), wherein the frame (31) of the seat is connected to the chassis (22) by a positioning arm (32), which is at its upper end rotationally connected to the frame (31) of the seat and at its lower end it is rotationally connected to the chassis (22), wherein the rotational connection (33) of the positioning arm (32) to the chassis (22) is located at the front third of the length of the chassis (22), wherein the wheelchair (1) further comprises a positioning linear drive (34) for tilting the positioning arm (32) relative to the chassis (22), wherein the positioning linear drive (34) is connected at one end to the chassis (22) and at the other end it is connected to the positioning arm (32), wherein the connection of the positioning linear drive (34) to the chassis (22) is located closer to the back wheels (24) than the rotational connection (33) of the positioning arm (32) to the chassis (22).

9. The assembly for transporting persons with disability according to claim 8, **characterized in that** the positioning arm (32) comprises a parallelogram comprising two profiles (35) of the arm offset from each other in the longitudinal direction of the wheelchair (1), wherein each profile (35) of the arm is rotationally connected to the chassis (22) and to the frame (31) of the seat.

10. The assembly for transporting persons with disability according to any one of claims 8 or 9, **characterized in that** the rotational connection of the positioning arm (32) to the chassis (22) is located between the front wheels (23) of the wheelchair (1).

11. The assembly for transporting persons with disability according to any one of claims 8 to 10, **characterized in that** the seat (25) is adjustable by the positioning linear drive (34) to two extreme positions, wherein the lowest positioned point of the seating area of the seat (25) is in the first position a maximum of 35 cm higher than the lowest positioned point of the wheels, and in the second position it is at least 45 cm higher than the lowest positioned point of the wheels.

12. The assembly for transporting persons with disability according to any one of claims 8 to 11, **characterized in that** the frame (31) of the seat comprises a top piece (36) and a bottom piece (37) which are movably connected to each other, wherein the bottom piece (37) is connected to the positioning arm (32), the top piece (36) supports the seating area and the top piece (36) is tiltable relative to the bottom piece (37).

13. The assembly for transporting persons with disability according to any one of claims 8 to 12, **characterized in that** the movement of the backrest (26) relative to the seat (25) and/or the footrest (27) relative to the seat (25) and/or the top piece (36) of the frame (31) of the seat relative to the bottom piece (37) of the frame (31) of the seat is electronically or mechanically coupled to the movement of the positioning linear drive (34).

14. A passenger automobile (2) **characterized in that** it comprises the assembly according to any one of the preceding claims, wherein the support column (3) is attached to the floor of the automobile (2).

15. A method for transporting a wheelchair (1) for transporting persons with disability into an automobile using the assembly according to any preceding claim, **characterized in that**
• a door (18) of the automobile (2) is opened, subsequently
• the wheelchair (1) is driven to the open door (18), wherein the back side of the wheelchair (1) faces the automobile (2), and, before or after the approach of the wheelchair (1), an arm (4) attached slidably in a vertical and horizontal direction to a support column (3) fixed to the floor of the automobile (2) and attached rotationally relative to the floor of the automobile (2) is extended from the open door (18), wherein the wheelchair (1) is connected to the arm (4) via an attachment (5),
• wherein the arm (4) is attached to the support column (3) via a connecting element (13) which moves together with the arm (4) when the arm (4) slides vertically, wherein the arm (4) is slidable horizontally relative to the connecting element (13), and subsequently
• the wheelchair (1) is transferred by the arm (4) to the automobile (2), wherein the transfer comprises at least lifting the wheelchair (1), transferring the wheelchair (1) closer to the support column (3), and rotating the wheelchair (1).

## Patentansprüche

1. Eine Anordnung zum Transport eines Rollstuhls (1) zum Transport von Personen mit Behinderung in und aus einem Kraftwagen (2), umfassend eine Stützsäule (3) zur Befestigung an einem Boden des Kraftwagens (2), einen mit der Stützsäule (3) verbundenen Arm (4), und einen Antriebsmechanismus zum Bewegen des Arms (4), wobei der Arm (4) mit einer Befestigung (5) zum Befestigen des Rollstuhls (1) am Arm (4) versehen ist, **dadurch gekennzeichnet, dass** der Antriebsmechanismus ein Hubführungselement (6) mit einem Hubantrieb (7) zum vertikalen Verschieben des Arms (4) relativ zur Stützsäule (3), ein Schiebeführungselement (8) mit einem Schiebeantrieb (9) zum horizontalen Verschieben des Arms (4) relativ zur Stützsäule (3), um den Drehradius des Rollstuhls (1) um eine Achse der Stützsäule (3) zu verändern, wenn der Rollstuhl (1) an der Befestigung (5) befestigt ist, und ein Drehführungselement (10) mit einem Drehantrieb (11) zum Drehen des Arms (4) um die Achse der Stützsäule (3) umfasst, wobei in Richtung der Achse der Stützsäule (3) gesehen die Achse des Schiebeführungselements (8) gegenüber der Stützsäule (3) versetzt ist, wobei der Antriebsmechanismus ferner eine Steuereinheit (12) umfasst, die zum Betreiben des Hubantriebs (7), des Schiebeantriebs (9) und des Drehantriebs (11) ausgelegt ist, wobei der Antriebsmechanismus ein Verbindungselement (13) umfasst, das verschiebbar an der Stützsäule (3) befestigt ist und sich zusammen mit dem Arm (4) bewegt, wenn der Arm (4) vertikal verschoben wird, wobei der Arm (4) verschiebbar am Verbindungselement (13) befestigt ist und der Schiebeantrieb (9) dazu ausgelegt ist, den Arm (4) horizontal relativ zu dem Verbindungselement (13) zu verschieben.

2. Die Anordnung zum Transport eines Rollstuhls (1) zum Transport von Personen mit Behinderung gemäß Anspruch 1, **dadurch gekennzeichnet, dass** sowohl das Hubführungselement (6) als auch das Schiebeführungselement (8) durch das Verbindungselement (13) verlaufen.

3. Die Anordnung zum Transport eines Rollstuhls (1) zum Transport von Personen mit Behinderung gemäß einem der vorstehenden Ansprüche, **dadurch gekennzeichnet, dass** das Schiebeführungselement (8), in der Achse der Stützsäule (3) gesehen, neben der Stützsäule (3) verläuft.

4. Die Anordnung zum Transport eines Rollstuhls (1) zum Transport von Personen mit Behinderung gemäß einem der vorstehenden Ansprüche, **dadurch gekennzeichnet, dass** die Steuereinheit (12) dazu ausgelegt ist, mindestens zwei Antriebe aus dem Hubantrieb (7), dem Schiebeantrieb (9) und dem Drehantrieb (11) gleichzeitig zu steuern.

5. Die Anordnung zum Transport eines Rollstuhls (1) zum Transport von Personen mit Behinderung gemäß einem der vorstehenden Ansprüche, **dadurch gekennzeichnet, dass** zum Transport des Rollstuhls (1) in den Kraftwagen (2) nach dem Befestigen des Rollstuhls (1) an der Befestigung (5), die Steuereinheit (12) so ausgelegt ist, dass sie die Antriebe zum Anheben des Rollstuhls (1), zum Annähern des Rollstuhls (1) an die Stützsäule (3), zum Drehen des Rollstuhls (1) um die Säule (3), zum Entfernen des Rollstuhls (1) von der Stützsäule (3) und zum Abstellen des Rollstuhls (1) auf dem Boden des Kraftwagens (2) steuert.

6. Die Anordnung zum Transport eines Rollstuhls (1) zum Transport von Personen mit Behinderung gemäß einem der vorstehenden Ansprüche, **dadurch gekennzeichnet, dass** sie ferner einen Mechanismus (17) zum Blockieren der Bewegung der Tür (18) des Kraftwagens (2) umfasst.

7. Die Anordnung zum Transport eines Rollstuhls (1) zum Transport von Personen mit Behinderung gemäß einem der vorstehenden Ansprüche, **dadurch gekennzeichnet, dass** sie ferner einen Hilfsrahmen zum Befestigen am Rollstuhl (1) umfasst, wobei die Befestigung (5) zum Befestigen am Hilfsrahmen ausgelegt ist.

8. Eine Anordnung zum Transport von Personen mit Behinderung, die die Anordnung zum Transport eines Rollstuhls (1) gemäß einem der vorstehenden Ansprüche umfasst, **dadurch gekennzeichnet, dass** sie ferner einen Rollstuhl (1) zum Transport von Personen mit Behinderung umfasst, der ein mit einem Paar Vorderrädern (23) und einem Paar Hinterrädern (24) versehenes Fahrgestell (22), einen Sitz (25), eine Rückenlehne (26), eine Fußstütze (27), eine Kraftquelle (28) und einen Fahrantrieb (29) umfasst, wobei die Rückenlehne (26) einen Rückenlehnerahmen (30) umfasst und der Sitz (25) einen Sitzrahmen (31) umfasst, der beweglich mit dem Rückenlehnerahmen (30), der Fußstütze (27) und dem Fahrgestell (22) verbunden ist, wobei das Paar Vorderrädern (23) durch das Fahrgestell (22) in einem konstanten Abstand zum Paar Hinterrädern (24) gehalten wird, wobei der Sitzrahmen (31) mit dem Fahrgestell (22) durch einen Positionierungsarm (32) verbunden ist, der an seinem oberen Ende drehbar mit dem Sitzrahmen (31) verbunden ist und an seinem unteren Ende drehbar mit dem Fahrgestell (22) verbunden ist, wobei sich die Drehverbindung (33) des Positionierungsarms (32) mit dem Fahrgestell (22) im vorderen Drittel der Länge des Fahrgestells (22) befindet, wobei der Rollstuhl (1) ferner einen Positionierungslinearantrieb (34) zum Neigen des Positionierungsarms (32) relativ zum Fahrgestell (22) umfasst, wobei der Positionierungslinearantrieb (34) an einem Ende mit dem Fahrgestell (22) und am anderen Ende mit dem Positionierungsarm (32) verbunden ist, wobei die Verbindung des Positionierungslinearantriebs (34) mit dem Fahrgestell (22) näher an den Hinterrädern (24) angeordnet ist als die Drehverbindung (33) des Positionierungsarms (32) mit dem Fahrgestell (22).

9. Die Anordnung zum Transport von Personen mit Behinderung gemäß Anspruch 8, **dadurch gekennzeichnet, dass** der Positionierungsarm (32) ein Parallelogramm umfasst, das zwei Profile (35) des Arms umfasst, die in Längsrichtung des Rollstuhls (1) versetzt zueinander angeordnet sind, wobei jedes Profil (35) des Arms drehbar mit dem Fahrgestell (22) und mit dem Sitzrahmen (31) verbunden ist.

10. Die Anordnung zum Transport von Personen mit Behinderung gemäß einem der Ansprüche 8 oder 9, **dadurch gekennzeichnet, dass** die Drehverbindung des Positionierungsarms (32) mit dem Fahrgestell (22) zwischen den Vorderrädern (23) des Rollstuhls (1) angeordnet ist.

11. Die Anordnung zum Transport von Personen mit Behinderung gemäß einem der Ansprüche 8 bis 10, **dadurch gekennzeichnet, dass** der Sitz (25) durch den Positionierungslinearantrieb (34) in zwei Endpositionen verstellbar ist, wobei der tiefste Punkt der Sitzfläche des Sitzes (25) in der ersten Position maximal 35 cm höher als der tiefste Punkt der Räder liegt und in der zweiten Position mindestens 45 cm höher als der tiefste Punkt der Räder liegt.

12. Die Anordnung zum Transport von Personen mit Behinderung gemäß einem der Ansprüche 8 bis 11, **dadurch gekennzeichnet, dass** der Sitzrahmen (31) ein Oberteil (36) und ein Unterteil (37) umfasst, die beweglich miteinander verbunden sind, wobei das Unterteil (37) mit dem Positionierungsarm (32) verbunden ist, das Oberteil (36) die Sitzfläche trägt und das Oberteil (36) relativ zum Unterteil (37) kippbar ist.

13. Die Anordnung zum Transport von Personen mit Behinderung gemäß einem der Ansprüche 8 bis 12, **dadurch gekennzeichnet, dass** die Bewegung der Rückenlehne (26) relativ zum Sitz (25) und/oder der Fußstütze (27) relativ zum Sitz (25) und/oder des Oberteils (36) des Sitzrahmens (31) relativ zum Unterteil (37) des Sitzrahmens (31) elektronisch oder mechanisch mit der Bewegung des Positionierungslinearantrieb (34) gekoppelt ist.

14. Ein Personenkraftwagen (2), **dadurch gekennzeichnet, dass** er die Anordnung gemäß einem der vorstehenden Ansprüche umfasst, wobei die Stützsäule (3) am Boden des Kraftwagens (2) befestigt ist.

15. Ein Verfahren zum Transport eines Rollstuhls (1) zum Transport von Personen mit Behinderung in einen Kraftwagen unter Verwendung der Anordnung gemäß einem der vorstehenden Ansprüche, **dadurch gekennzeichnet, dass**
• eine Tür (18) des Kraftwagens (2) geöffnet wird, anschließend
• der Rollstuhl (1) zur offenen Tür (18) gefahren wird, wobei die Rückseite des Rollstuhls (1) zum Kraftwagen (2) zeigt, und vor oder nach dem Heranfahren des Rollstuhls (1) ein in vertikaler und horizontaler Richtung verschiebbar an einer am Boden des Kraftwagens (2) befestigten Stützsäule (3) befestigter und relativ zum Boden des Kraftwagens (2) drehbar gelagerter Arm (4) aus der geöffneten Tür (18) herausgefahren wird, wobei der Rollstuhl (1) über eine Befestigung (5) mit dem Arm (4) verbunden ist,
• wobei der Arm (4) über ein Verbindungselement (13) an der Stützsäule (3) befestigt ist, das sich zusammen mit dem Arm (4) bewegt, wenn sich der Arm (4) vertikal verschiebt, wobei der Arm (4) relativ zum Verbindungselement (13) horizontal verschiebbar ist, und anschließend
• der Rollstuhl (1) durch den Arm (4) in den Kraftwagen (2) übertragen wird, wobei die Übertragung mindestens das Anheben des Rollstuhls (1), das Annähern des Rollstuhls (1) an die Stützsäule (3) und das Drehen des Rollstuhls (1) umfasst.

## Revendications

1. Un ensemble de transport d'un fauteuil roulant (1) pour le transport de personnes en situation de handicap vers et depuis une automobile (2) comprenant une colonne de support (3) destinée à être fixée au plancher de l'automobile (2), un bras (4) relié à la colonne de support (3), et un mécanisme d'entraînement pour déplacer le bras (4), où le bras (4) est muni d'une fixation (5) pour fixer le fauteuil roulant (1) au bras (4), **caractérisé en ce que** le mécanisme d'entraînement comprend un élément de guidage de levage (6) avec un entraînement de levage (7) pour faire coulisser verticalement le bras (4) par rapport à la colonne de support (3), un élément de guidage coulissant (8) avec un entraînement coulissant (9) pour faire coulisser horizontalement le bras (4) par rapport à la colonne de support (3) afin de modifier le rayon de rotation du fauteuil roulant (1) autour d'un axe de la colonne de support (3) lorsque le fauteuil roulant (1) est fixé à la fixation (5), et un élément de guidage rotatif (10) avec un entraînement rotatif (11) pour faire tourner le bras (4) autour de l'axe de la colonne de support (3), où, vu dans la direction de l'axe de la colonne de support (3), l'axe de l'élément de guidage coulissant (8) est décalé par rapport à la colonne de support (3), où le mécanisme d'entraînement comprend en outre une unité de commande (12) adaptée pour actionner l'entraînement de levage (7), l'entraînement coulissant (9) et l'entraînement rotatif (11), où le mécanisme d'entraînement comprend un élément de liaison (13) qui est fixé de manière coulissante sur la colonne de support (3) et se déplace avec le bras (4) lorsque le bras (4) coulisse verticalement, où le bras (4) est fixé de manière coulissante à l'élément de liaison (13) et l'entraînement coulissant (9) est adapté pour faire coulisser le bras (4) horizontalement par rapport à l'élément de liaison (13).

2. L'ensemble de transport d'un fauteuil roulant (1) pour le transport de personnes en situation de handicap selon la revendication 1, **caractérisé en ce que** l'élément de guidage de levage (6) et l'élément de guidage coulissant (8) passent tous deux à travers l'élément de liaison (13).

3. L'ensemble de transport d'un fauteuil roulant (1) pour le transport de personnes en situation de handicap selon l'une quelconque des revendications précédentes, **caractérisé en ce que,** vu dans l'axe de la colonne de support (3), l'élément de guidage coulissant (8) passe à côté de la colonne de support (3).

4. L'ensemble de transport d'un fauteuil roulant (1) pour le transport de personnes en situation de handicap selon l'une quelconque des revendications précédentes, **caractérisé en ce que** l'unité de commande (12) est adaptée pour commander simultanément au moins deux entraînements parmi l'entraînement de levage (7), l'entraînement coulissant (9) et l'entraînement rotatif (11).

5. L'ensemble de transport d'un fauteuil roulant (1) pour le transport de personnes en situation de handicap selon l'une quelconque des revendications précédentes, **caractérisé en ce que** pour transporter le fauteuil roulant (1) dans l'automobile (2) après avoir fixé le fauteuil roulant (1) à la fixation (5), l'unité de commande (12) est adaptée pour commander les entraînements afin de soulever le fauteuil roulant (1), de rapprocher le fauteuil roulant (1) de la colonne de support (3), de faire pivoter le fauteuil roulant (1) autour de la colonne, d'éloigner le fauteuil roulant (1) de la colonne de support (3) et de placer le fauteuil roulant (1) sur le plancher de l'automobile (2).

6. L'ensemble de transport d'un fauteuil roulant (1) pour le transport de personnes en situation de handicap selon l'une quelconque des revendications précédentes, **caractérisé en ce qu'**il comprend en outre un mécanisme (17) pour bloquer le mouvement de la porte (18) de l'automobile (2).

7. L'ensemble de transport d'un fauteuil roulant (1) pour le transport de personnes en situation de handicap selon l'une quelconque des revendications précédentes, **caractérisé en ce qu'**il comprend en outre un cadre auxiliaire destiné à être fixé au fauteuil roulant (1), où la fixation (5) est adaptée pour être fixée au cadre auxiliaire.

8. Un ensemble pour le transport de personnes en situation de handicap comprenant l'ensemble pour le transport d'un fauteuil roulant (1) selon l'une quelconque des revendications précédentes, **caractérisé en ce qu'**il comprend en outre un fauteuil roulant (1) pour le transport de personnes en situation de handicap, comprenant un châssis (22) muni d'une paire de roues avant (23) et d'une paire de roues arrière (24), un siège (25), un dossier (26), un repose-pieds (27), une source d'énergie (28) et un dispositif d'entraînement (29), où le dossier (26) comprend un cadre (30) du dossier et le siège (25) comprend un cadre (31) du siège relié de manière mobile au cadre (30) du dossier, au repose-pieds (27) et au châssis (22), où la paire de roues avant (23) est maintenue à une distance constante de la paire de roues arrière (24) par le châssis (22), où le cadre (31) du siège est relié au châssis (22) par un bras de positionnement (32) qui est relié de manière rotative à son extrémité supérieure au cadre (31) du siège et à son extrémité inférieure au châssis (22), où la liaison rotative (33) du bras de positionnement (32) au châssis (22) est située dans le tiers avant de la longueur du châssis (22), où le fauteuil roulant (1) comprend en outre un entraînement linéaire de positionnement (34) pour incliner le bras de positionnement (32) par rapport au châssis (22), où l'entraînement linéaire de positionnement (34) est relié à une extrémité au châssis (22) et à l'autre extrémité au bras de positionnement (32), où la liaison de l'entraînement linéaire de positionnement (34) au châssis (22) est située plus près des roues arrière (24) que la liaison rotative (33) du bras de positionnement (32) au châssis (22).

9. L'ensemble pour le transport de personnes en situation de handicap selon la revendication 8, **caractérisé en ce que** le bras de positionnement (32) comprend un parallélogramme comprenant deux profilés (35) du bras, décalés l'un par rapport à l'autre dans la direction longitudinale du fauteuil roulant (1), où chaque profilé (35) du bras est relié de manière rotative au châssis (22) et au cadre (31) du siège.

10. L'ensemble pour le transport de personnes en situation de handicap selon l'une quelconque des revendications 8 ou 9, **caractérisé en ce que** la liaison rotative du bras de positionnement (32) au châssis (22) est située entre les roues avant (23) du fauteuil roulant (1).

11. L'ensemble pour le transport de personnes en situation de handicap selon l'une quelconque des revendications 8 à 10, **caractérisé en ce que** le siège (25) est réglable par l'entraînement linéaire de positionnement (34) sur deux positions extrêmes, où le point le plus bas de la zone d'assise du siège (25) est, dans la première position, au maximum 35 cm plus haut que le point le plus bas des roues, et dans la deuxième position, il est au moins 45 cm plus haut que le point le plus bas des roues.

12. L'ensemble pour le transport de personnes en situation de handicap selon l'une quelconque des revendications 8 à 11, **caractérisé en ce que** le cadre (31) du siège comprend une partie supérieure (36) et une partie inférieure (37) qui sont reliées de manière mobile l'une à l'autre, où la partie inférieure (37) est reliée au bras de positionnement (32), la partie supérieure (36) supporte la zone d'assise et la partie supérieure (36) est inclinable par rapport à la partie inférieure (37).

13. L'ensemble pour le transport de personnes en situation de handicap selon l'une quelconque des revendications 8 à 12, **caractérisé en ce que** le mouvement du dossier (26) par rapport au siège (25) et/ou du repose-pieds (27) par rapport au siège (25) et/ou de la partie supérieure (36) du cadre (31) du siège par rapport à la partie inférieure (37) du cadre (31) du siège est couplé électroniquement ou mécaniquement au mouvement de l'entraînement linéaire de positionnement (34).

14. Une automobile particulière (2) **caractérisée en ce qu'**elle comprend l'ensemble selon l'une quelconque des revendications précédentes, où la colonne de support (3) est fixée au plancher de l'automobile (2).

15. Un procédé de transport d'un fauteuil roulant (1) pour le transport de personnes en situation de handicap dans une automobile à l'aide de l'ensemble selon l'une quelconque des revendications précédentes, **caractérisé en ce que**
• une porte (18) de l'automobile (2) est ouverte, puis
• le fauteuil roulant (1) est conduit vers la porte ouverte (18), où la partie arrière du fauteuil roulant (1) fait face à l'automobile (2), et, avant ou après l'approche du fauteuil roulant (1), un bras (4) fixé de manière coulissante dans le sens vertical et horizontal à une colonne de support (3) fixée au plancher de l'automobile (2) et fixé de manière rotative par rapport au plancher de l'automobile (2) est déployé depuis la porte ouverte (18), où le fauteuil roulant (1) est relié au bras (4) via une fixation (5),
• où le bras (4) est fixé à la colonne de support (3) via un élément de liaison (13) qui se déplace avec le bras (4) lorsque le bras (4) coulisse verticalement, où le bras (4) peut coulisser horizontalement par rapport à l'élément de liaison (13), et puis
• le fauteuil roulant (1) est transféré par le bras (4) vers l'automobile (2), où le transfert comprend au moins le levage du fauteuil roulant (1), le rapprochement du fauteuil roulant (1) de la colonne de support (3) et la rotation du fauteuil roulant (1).
